# EUROPEAN PATENT APPLICATION

(11) **EP 4 785 946 A1**
(43) Date of publication of application: **05.08.2026**
(21) Application number: 24870859.6
(22) Date of filing: 26.09.2024
(51) Int. Cl.: A61K 31/713, A61K 47/54, C12N 15/113, A61P 35/00

(54) **LIPID-MODIFIED NUCLEIC ACID COMPOUND, AND PREPARATION METHOD AND USE THEREFOR**

(30) Priority: 26.09.2023 CN 202311251626
(71) Applicant: Tuojie Biotech (Shanghai) Co., Ltd., Shanghai 201203 (CN)
(72) Inventor: TAN, Liang, Shanghai 201203 (CN); LI, Yunfei, Shanghai 201203 (CN)
(74) Representative: Regimbeau
(86) International application number: PCT/CN2024/121360
(87) International publication number: WO 2025/067317

(57) **Abstract**

The present invention relates to a lipid-modified nucleic acid compound, and a preparation method and use therefor, and specifically relates to a conjugate as represented by formula I or a pharmaceutically acceptable salt thereof.

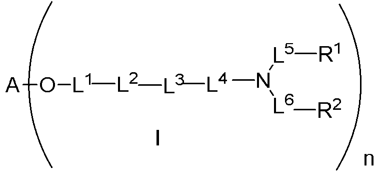

## Description

### TECHNICAL FIELD

The present disclosure relates to the field of pharmaceuticals and specifically relates to a lipid-modified nucleic acid compound, a preparation method therefor, and use thereof.

### BACKGROUND

RNA interference (RNAi) is an effective way of silencing gene expression and has wide application prospects. While RNA interference drugs have advantages such as a broad selection of targets, a simple and efficient development process, and high specificity, the number of RNA interference drugs approved by the FDA for clinical treatment remains very limited. There are several major pharmacological challenges, including poor stability, short half-life, high immunogenicity, small targeting capacity, limited cytoplasmic delivery, etc. Therefore, in addition to improving the stability and immunogenicity of RNA interference drugs through chemical modifications, providing an efficient and safe *in vivo* delivery system and improving membrane permeability are also needed.

The delivery technologies for RNA interference drugs mainly include lipid nanoparticle (LNP) delivery, liver-targeted delivery mediated by N-acetylgalactosamine (GalNAC) modification, targeted delivery based on antibody mediation, etc. GalNAC-conjugated RNA drugs enable efficient and specific delivery to the liver. However, for extrahepatic cells that lack ASGPR expression, effective delivery methods are still unavailable. Therefore, there is a need to continue to develop efficient *in vivo* delivery methods to enable RNA drugs to exert their effects in extrahepatic tissues.

### SUMMARY

The present disclosure provides a conjugate represented by formula I or a pharmaceutically acceptable salt thereof, 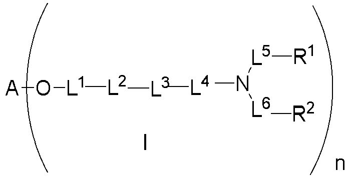
wherein A is a nucleic acid;
L¹, L², L³, and L⁴ are each independently a bond, -O-, -S-, -NH-, -C(=O)-, -OC(=O)-, - C(=O)O-, -NHC(=O)-, -C(=O)NH-, or unsubstituted or R³-substituted C₁₋₁₀ alkylene;
R³ is -OH;
L⁵ is -L^{5A}-L^{5B}-L^{5C}-L^{5D}-;
L⁶ is -L^{6A}-L^{6B}-L^{6C-}L^{6D}-;
L^{5A}, L^{5B}, L^{5C}, L^{5D}, L^{6A}, L^{6B}, L^{6C}, and L^{6D} are each independently a bond, -O-, -NH-, - C(=O)-, -OC(=O)-, -C(=O)O-, -NHC(=O)-, -C(=O)NH-, -(CH₂)ₘ₁-, unsubstituted or R⁴-substituted 3-10 membered heteroaryl, or unsubstituted or R⁹-substituted 6-14 membered aryl;
R⁴ and R⁹ are each independently -OH, -NH₂, -CN, halogen, C₁₋₄ alkyl, C₁₋₄ alkoxy, or C₁₋₄ haloalkyl;
R¹ and R² are each independently unsubstituted saturated or unsaturated C₁₋₂₅ hydrocarbyl, or saturated or unsaturated C₁₋₂₅ hydrocarbyl substituted with one or more R¹⁰;
each R¹⁰ is independently halogen or 3-6 membered cycloalkyl;
or when a carbon atom has two R¹⁰ substituents, the two R¹⁰ substituents, together with the carbon atom to which they are attached, form 3-6 membered cycloalkyl;
m1 is 1, 2, 3, 4, 5, 6, 7, 8, 9, or 10;
n is 1, 2, 3, 4, 5, or 6.

In some embodiments, only the following substituents or groups are limited, and the substituents or groups not involved are defined as described in any one of the other embodiments (hereinafter referred to as "in some embodiments");
A is a nucleic acid;
L¹, L², L³, and L⁴ are each independently a bond, -NH-, -C(=O)-, -NHC(=O)-, -C(=O)NH-, or unsubstituted or R³-substituted C₁₋₁₀ alkyl;
R³ is -OH;
L⁵ is -L^{5A}-L^{5B}-L^{5C}-L^{5D}-;
L⁶ is -L^{6A}-L^{6B}-L^{6C-}L^{6D}-;
L^{5A}, L^{5B}, L^{5C}, L^{5D}, L^{6A}, L^{6B}, L^{6C}, and L^{6D} are each independently a bond, -O-, -NH-, - C(=O)-, -NHC(=O)-, -C(=O)NH-, -(CH₂)ₘ₁-, unsubstituted 3-10 membered heteroaryl, or unsubstituted 6-14 membered aryl;
R¹ and R² are each independently unsubstituted saturated C₁₋₂₅ hydrocarbyl, unsubstituted unsaturated C₃₋₂₅ hydrocarbyl containing 1-10 carbon-carbon double bonds, or saturated C₁₋₂₅ hydrocarbyl substituted with one or more R¹⁰;
each R¹⁰ is independently halogen or 3-6 membered cycloalkyl;
or when a carbon atom has two R¹⁰ substituents, the two R¹⁰ substituents, together with the carbon atom to which they are attached, form 3-6 membered cycloalkyl;
m1 is 1, 2, 3, 4, 5, 6, 7, 8, 9, or 10;
n is 1, 2, 3, 4, 5, or 6.

In some embodiments, in L¹, L², L³, and L⁴, the C₁₋₁₀ alkyl is C₁ alkyl, C₂ alkyl, C₃ alkyl, C₄ alkyl, C₅ alkyl, C₆ alkyl, C₇ alkyl, C₈ alkyl, C₉ alkyl, or C₁₀ alkyl.

In some embodiments, in L¹, L², L³, and L⁴, the C₁₋₁₀ alkyl is C₄₋₈ alkyl.

In some embodiments, in L¹, L², L³, and L⁴, the C₁₋₁₀ alkyl is C₁₋₄ alkyl.

In some embodiments, in L¹, L², L³, and L⁴, the C₁₋₁₀ alkyl is linear or branched C₁₋₁₀ alkyl.

In some embodiments, L¹, L², L³, and L⁴ are each independently a bond, -NH-, -C(=O)-, -NHC(=O)-, -C(=O)NH-, or unsubstituted or R³-substituted C₁₋₁₀ alkyl.

In some embodiments, -L¹-L²-L³-L⁴- is -R³-substituted C₁₋₁₀ alkyl-C(=O)- or -R³-substituted C₁₋₁₀ alkyl-. In some embodiments, the 3' end of a nucleic acid strand of the nucleic acid is linked to -O-L¹-.

In some embodiments, -L¹-L²-L³-L⁴- is -R³-substituted C₄₋₈ alkyl-C(=O)- or -R³-substituted C₄₋₈ alkyl-. In some embodiments, the 3' end of a nucleic acid strand of the nucleic acid is linked to -O-L¹-.

In some embodiments, -L¹-L²-L³-L⁴- is -R³-substituted C₅ alkyl-C(=O)-, -R³-substituted C₆ alkyl-C(=O)-, -R³-substituted C₇ alkyl-C(=O)-, -R³-substituted C₈ alkyl-C(=O)-, -R³-substituted C₅ alkyl-, -R³-substituted C₆ alkyl-, -R³-substituted C₇ alkyl-, or -R³-substituted C₈ alkyl-. In some embodiments, the 3' end of a nucleic acid strand of the nucleic acid is linked to -O-L¹-.

In some embodiments, L¹, L², L³, and L⁴ are each independently a bond, -NH-, -C(=O)-, -NHC(=O)-, -C(=O)NH-, -(CH₂)ₘ₂-, or -(CH₂)ₘ₃-CH(CH₂OH)-(CH₂)ₘ₄-, wherein m2, m3, and m4 are each independently 1, 2, 3, 4, 5, 6, 7, 8, 9, or 10. In some embodiments, the 3' end of a nucleic acid strand of the nucleic acid is linked to -O-L¹-.

In some embodiments, -L¹-L²-L³-L⁴- is -(CH₂)ₘ₃-CH(CH₂OH)-(CH₂)ₘ₄-C(=O)- or - (CH₂)ₘ₃-CH(CH₂OH)-(CH₂)ₘ₄-, wherein m3 and m4 are each independently 1, 2, 3, 4, 5, 6, 7, 8, 9, or 10. In some embodiments, the 3' end of a nucleic acid strand of the nucleic acid is linked to -O-L¹-.

In some embodiments, -L¹-L²-L³-L⁴- is 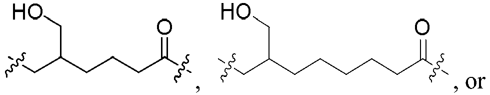 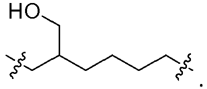 In some embodiments, the 3' end of a nucleic acid strand of the nucleic acid is linked to -O-L¹-.

In some embodiments, the number of R³ is 1, 2, or 3.

In some embodiments, -L¹-L²-L³-L⁴- is -unsubstituted C₁₋₁₀ alkyl-C(=O)- or - unsubstituted C₁₋₁₀ alkyl-. In some embodiments, the 5' end of a nucleic acid strand of the nucleic acid is linked to -O-L¹-.

In some embodiments, -L¹-L²-L³-L⁴- is -unsubstituted C₄₋₈ alkyl-C(=O)- or -unsubstituted C₄₋₈ alkyl-. In some embodiments, the 5' end of a nucleic acid strand of the nucleic acid is linked to -O-L¹-.

In some embodiments, -L¹-L²-L³-L⁴- is -unsubstituted C₅ alkyl-C(=O)-, -unsubstituted C₆ alkyl-C(=O)-, -unsubstituted C₇ alkyl-C(=O)-, -unsubstituted C₈ alkyl-C(=O)-, - unsubstituted C₅ alkyl-, -unsubstituted C₆ alkyl-, -unsubstituted C₇ alkyl-, or - unsubstituted C₈ alkyl-. In some embodiments, the 5' end of a nucleic acid strand of the nucleic acid is linked to -O-L¹-.

In some embodiments, -L¹-L²-L³-L⁴- is -(CH₂)ₘ₂-C(=O)- or -(CH₂)ₘ₂-, wherein m2 is independently 1, 2, 3, 4, 5, 6, 7, 8, 9, or 10. In some embodiments, the 5' end of a nucleic acid strand of the nucleic acid is linked to -O-L¹-.

In some embodiments, -L¹-L²-L³-L⁴- is 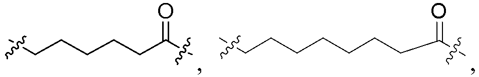 or 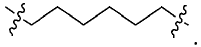 In some embodiments, the 5' end of a nucleic acid strand of the nucleic acid is linked to -O-L¹-.

In some embodiments, in L^{5A}, L^{5B}, L^{5C}, L^{5D}, L^{6A}, L^{6B}, L^{6C}, and L^{6D}, the 3-10 membered heteroaryl is 3-10 membered heteroaryl containing 1, 2, or 3 heteroatoms selected from the group consisting of N, O, and S.

In some embodiments, in L^{5A}, L^{5B}, L^{5C}, L^{5D}, L^{6A}, L^{6B}, L^{6C}, and L^{6D}, the 3-10 membered heteroaryl is 3-8 membered heteroaryl containing 1, 2, or 3 heteroatoms selected from the group consisting of N, O, and S.

In some embodiments, in L^{5A}, L^{5B}, L^{5C}, L^{5D}, L^{6A}, L^{6B}, L^{6C}, and L^{6D}, the 3-10 membered heteroaryl is 3-6 membered heteroaryl containing 1, 2, or 3 heteroatoms selected from the group consisting of N, O, and S.

In some embodiments, in L^{5A}, L^{5B}, L^{5C}, L^{5D}, L^{6A}, L^{6B}, L^{6C}, and L^{6D}, the 3-10 membered heteroaryl is 5-6 membered heteroaryl containing 1, 2, or 3 heteroatoms selected from the group consisting of N and O.

In some embodiments, in L^{5A}, L^{5B}, L^{5C}, L^{5D}, L^{6A}, L^{6B}, L^{6C}, and L^{6D}, the 3-10 membered heteroaryl is 5-6 membered heteroaryl containing 1, 2, or 3 heteroatoms that are N.

In some embodiments, in L^{5A}, L^{5B}, L^{5C}, L^{5D}, L^{6A}, L^{6B}, L^{6C}, and L^{6D}, the 3-10 membered heteroaryl is triazolyl (e.g., 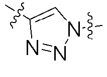 ).

In some embodiments, in L^{5A}, L^{5B}, L^{5C}, L^{5D}, L^{6A}, L^{6B}, L^{6C}, and L^{6D}, the 6-14 membered aryl is 6-10 membered aryl.

In some embodiments, in L^{5A}, L^{5B}, L^{5C}, L^{5D}, L^{6A}, L^{6B}, L^{6C}, and L^{6D}, the 6-14 membered aryl is phenyl (e.g., 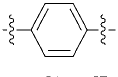 ).

In some embodiments, L^{5A}, L^{5B}, L^{5C}, L^{5D}, L^{6A}, L^{6B}, L^{6C}, and L^{6D} are each independently a bond, -O-, -NH-, -C(=O)-, -NHC(=O)-, -C(=O)NH-, -(CH₂)ₘ₁-, unsubstituted or R⁴-substituted 3-10 membered heteroaryl, or unsubstituted or R⁹-substituted 6-14 membered aryl, wherein the definitions and selection ranges of R⁴, R⁹, and m1 are as described in any one of the embodiments in the context of the present disclosure.

In some embodiments, L^{5A}, L^{5B}, L^{5C}, L^{5D}, L^{6A}, L^{6B}, L^{6C}, and L^{6D} are each independently a bond, -O-, -NH-, -C(=O)-, -NHC(=O)-, -C(=O)NH-, -(CH₂)ₘ₁-, unsubstituted 3-10 membered heteroaryl, or unsubstituted 6-14 membered aryl, wherein the definition and selection range of m1 are as described in any one of the embodiments in the context of the present disclosure.

In some embodiments, -L^{5A}-L^{5B}-L^{5C}-L^{5D}- is a bond, -(CH₂)ₘ₁-NHC(=O)-, -C(=O)-, - (CH₂)ₘ₁-unsubstituted 3-10 membered heteroaryl-, -(CH₂)ₘ₁-unsubstituted 6-14 membered aryl-, or -(CH₂)ₘ₁-O-unsubstituted 6-14 membered aryl-, wherein the definition and selection range of m1 are as described in any one of the embodiments in the context of the present disclosure.

In some embodiments, -L^{5A}-L^{5B}-L^{5C}-L^{5D}- is a bond, -(CH₂)ₘ₁-NHC(=O)-, -C(=O)-, - (CH₂)ₘ₁-unsubstituted 5-6 membered heteroaryl-, -(CH₂)ₘ₁-unsubstituted phenyl-, or - (CH₂)ₘ₁-O-unsubstituted phenyl-, wherein the definition and selection range of m1 are as described in any one of the embodiments in the context of the present disclosure.

In some embodiments, -L^{5A}-L^{5B}-L^{5C}-L^{5D}- is a bond, 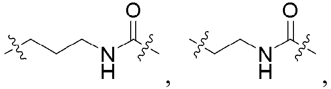 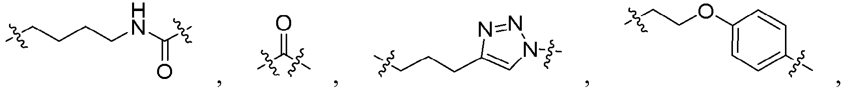 or

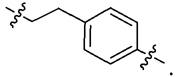

In some embodiments, -L^{6A}-L^{6B}-L^{6C-}L^{6D}- is a bond, -(CH₂)ₘ₁-NHC(=O)-, -C(=O)-, - (CH₂)ₘ₁-unsubstituted 3-10 membered heteroaryl-, -(CH₂)ₘ₁-unsubstituted 6-14 membered aryl-, or -(CH₂)ₘ₁-O-unsubstituted 6-14 membered aryl-, wherein the definition and selection range of m1 are as described in any one of the embodiments in the context of the present disclosure.

In some embodiments, -L^{6A}-L^{6B}-L^{6C-}L^{6D}- is a bond, -(CH₂)ₘ₁-NHC(=O)-, -C(=O)-, - (CH₂)ₘ₁-unsubstituted 5-6 membered heteroaryl-, -(CH₂)ₘ₁-unsubstituted phenyl-, or - (CH₂)ₘ₁-O-unsubstituted phenyl-, wherein the definition and selection range of m1 are as described in any one of the embodiments in the context of the present disclosure.

In some embodiments, -L^{6A}-L^{6B}-L^{6C-}L^{6D}- is a bond, 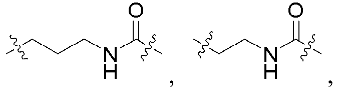 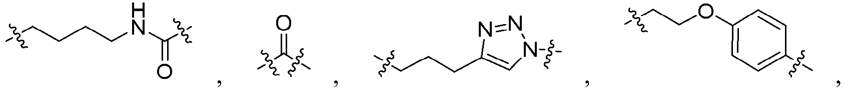 or

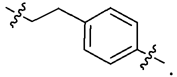

In some embodiments, L⁵ and L⁶ are identical.

In some embodiments, L⁵ and L⁶ are different.

In some embodiments, in R⁴ and R⁹, the halogen is fluorine, chlorine, or bromine.

In some embodiments, in R⁴ and R⁹, the C₁₋₄ alkyl is methyl, ethyl, n-propyl, isopropyl, n-butyl, isobutyl, sec-butyl, or tert-butyl.

In some embodiments, in R⁴ and R⁹, the C₁₋₄ alkoxy is methoxy, ethoxy, n-propoxy, isopropoxy, n-butoxy, isobutoxy, sec-butoxy, or tert-butoxy.

In some embodiments, in R⁴ and R⁹, the C₁₋₄ haloalkyl includes, but is not limited to, - CF₃, -CHF₂, or CH₂F.

In some embodiments, the numbers of R⁴ and R⁹ are independently 1, 2, or 3.

In some embodiments, in R¹⁰, the halogen is fluorine, chlorine, or bromine. In some embodiments, in R¹⁰, the halogen is fluorine.

In some embodiments, in R¹⁰, the 3-6 membered cycloalkyl is independently cyclopropyl, cyclobutyl, cyclopentyl, or cyclohexyl. In some embodiments, the 3-6 membered cycloalkyl is cyclopropyl or cyclobutyl. In some embodiments, the 3-6 membered cycloalkyl is cyclopropyl.

In some embodiments, the number of R¹⁰ is 1, 2, or 3. In some embodiments, the number of R¹⁰ is 1 or 2.

In some embodiments, in R¹ and R², the saturated or unsaturated C₁₋₂₅ hydrocarbyl is saturated C₁₋₂₅ hydrocarbyl, or unsaturated C₃₋₂₅ hydrocarbyl containing 1-10 carbon-carbon double bonds.

In some embodiments, the saturated C₁₋₂₅ hydrocarbyl is saturated C₆₋₂₅ hydrocarbyl. In some embodiments, in R¹ and R², the saturated C₁₋₂₅ hydrocarbyl is C₈₋₂₁ hydrocarbyl (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl).

In some embodiments, the unsaturated C₃₋₂₅ hydrocarbyl containing 1-10 carbon-carbon double bonds is unsaturated C₆₋₂₅ hydrocarbyl containing 1-10 carbon-carbon double bonds. In some embodiments, in R¹ and R², the unsaturated C₃₋₂₅ hydrocarbyl containing 1-10 carbon-carbon double bonds is unsaturated C₈₋₂₁ hydrocarbyl containing 1-8 carbon-carbon double bonds (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl). In some embodiments, in R¹ and R², the unsaturated C₃₋₂₅ hydrocarbyl containing 1-10 carbon-carbon double bonds is unsaturated C₈₋₂₁ hydrocarbyl containing 1-5 carbon-carbon double bonds (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl).

In some embodiments, in R¹ and R², the C₁₋₂₅ hydrocarbyl is linear or branched C₁₋₂₅ hydrocarbyl.

In some embodiments, the linear C₁₋₂₅ hydrocarbyl is linear C₃₋₂₅ hydrocarbyl. In some embodiments, the linear C₁₋₂₅ hydrocarbyl is linear C₆₋₂₅ hydrocarbyl. In some embodiments, the linear C₁₋₂₅ hydrocarbyl is linear C₈₋₂₁ hydrocarbyl (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl).

In some embodiments, the branched C₁₋₂₅ hydrocarbyl is branched C₃₋₂₅ hydrocarbyl. In some embodiments, the branched C₁₋₂₅ hydrocarbyl is branched C₆₋₂₅ hydrocarbyl. In some embodiments, the branched C₁₋₂₅ hydrocarbyl is branched C₈₋₂₁ hydrocarbyl (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl).

In some embodiments, R¹ and R² are each independently unsubstituted saturated C₁₋₂₅ hydrocarbyl, unsubstituted unsaturated C₃₋₂₅ hydrocarbyl containing 1-10 carbon-carbon double bonds, or saturated C₁₋₂₅ hydrocarbyl substituted with one or more R¹⁰.

In some embodiments, R¹ and R² are independently unsubstituted saturated C₆₋₂₅ hydrocarbyl, unsubstituted unsaturated C₆₋₂₅ hydrocarbyl containing 1-10 carbon-carbon double bonds, or saturated C₆₋₂₅ hydrocarbyl substituted with one or more R¹⁰.

In some embodiments, R¹ and R² are independently unsubstituted saturated C₈₋₂₁ hydrocarbyl (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl), unsubstituted unsaturated C₈₋₂₁ hydrocarbyl containing 1-8 carbon-carbon double bonds (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl), or saturated C₈₋₂₁ hydrocarbyl substituted with one or more R¹⁰ (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl).

In some embodiments, R¹ and R² are independently unsubstituted saturated C₈₋₂₁ hydrocarbyl (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl), unsubstituted unsaturated C₈₋₂₁ hydrocarbyl containing 1-5 carbon-carbon double bonds (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl), or saturated C₈₋₂₁ hydrocarbyl substituted with one or more R¹⁰ (e.g., C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, or C₂₁ hydrocarbyl).

In some embodiments, R¹ and R² are independently -(CH₂)₇CH₃, -(CH₂)₁₂CH₃, - (CH₂)₁₃CH₃, -(CH₂)₁₄CH₃, -(CH₂)₁₅CH₃, -(CH₂)₁₆CH₃, -(CH₂)₁₈CH₃, -(CH₂)₂₀CH₃,

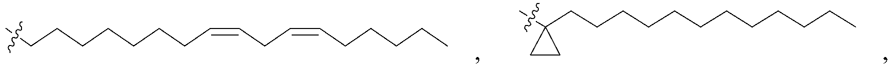

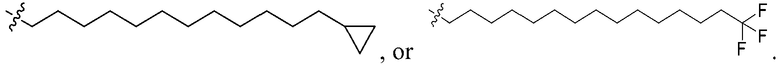

In some embodiments, R¹ and R² are identical.

In some embodiments, R¹ and R² are different.

In some embodiments, the conjugate represented by formula I is a conjugate represented by formula I-1 or I-2: 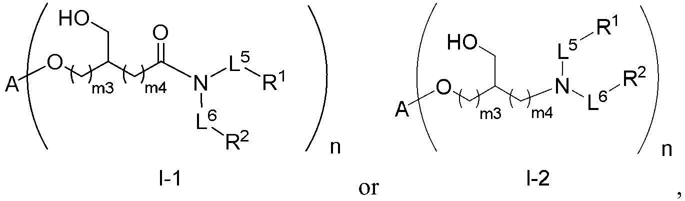 wherein m3 and m4 are each independently 1, 2, 3, 4, 5, 6, 7, 8, 9, or 10; the definitions and selection ranges of R¹, R², A, L⁵, L⁶, and n are as described in any one of the embodiments in the context of the present disclosure.

. In some embodiments, the 3' end of a nucleic acid strand of the nucleic acid is linked to -O-L¹-, and the definitions and selection ranges of L⁵, L⁶, R¹, and R² are as described in any one of the embodiments in the context of the present disclosure. In some embodiments, the conjugate represented by formula I is a conjugate represented by formula I-3 or I-4: 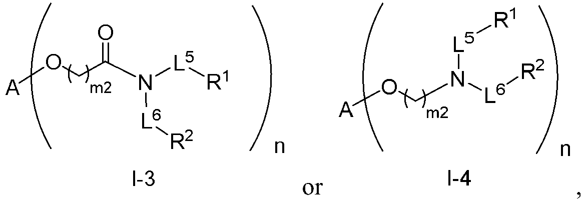 wherein m2 is 1, 2, 3, 4, 5, 6, 7, 8, 9, or 10; the definitions and selection ranges of R¹, R², A, L⁵, L⁶, and n are as described in any one of the embodiments in the context of the present disclosure. In some embodiments, the 5' end of a nucleic acid strand of the nucleic acid is linked to -O-L¹-, and the definitions and selection ranges of L⁵, L⁶, R¹, and R² are as described in any one of the embodiments in the context of the present disclosure.

In some embodiments, n is 1, 2, or 3.

In some embodiments, n is 1 or 2.

In some embodiments, n is 1.

In some embodiments, n is 2.

In some embodiments, n is 3.

In some embodiments, the nucleic acid includes, but is not limited to, a single-stranded oligonucleotide, a single-stranded antisense oligonucleotide, an siRNA, a dsRNA, an miRNA, an shRNA, and a Dicer substrate. In some examples, the nucleic acid further comprises a targeting ligand for targeting and mediating delivery to a position where a target mRNA is expressed, and the targeting ligand is covalently or non-covalently linked to a position in the nucleic acid other than the position linked to -O-L1-.

In some embodiments, the nucleic acid is a single-stranded nucleic acid.

In some embodiments, the single-stranded nucleic acid is the sense strand of an siRNA.

In some embodiments, the single-stranded nucleic acid is the antisense strand of an siRNA.

In some embodiments, the nucleic acid is a double-stranded nucleic acid.

In some embodiments, the nucleic acid is an siRNA. In some embodiments, the 3' end of the SS strand or the AS strand in the siRNA is linked to -O-L¹-. In some embodiments, the 5' end of the SS strand or the AS strand in the siRNA is linked to -O-L¹-. In some embodiments, both the 3' end and the 5' end of the SS strand or the AS strand in the siRNA are linked to -O-L¹-.

In some embodiments, the nucleic acid comprises one or more modified nucleotides.

In some embodiments, the nucleic acid is linked to L¹ by a phosphodiester group, wherein the O atom in -O-L¹- is attributed to the phosphodiester group.

In some embodiments, the nucleic acid is linked to L¹ by a phosphorothioate diester group, wherein the O atom in -O-L¹- is attributed to the phosphorothioate diester group.

In some embodiments, the 3' end of a nucleic acid strand of the nucleic acid is linked to - O-L¹-.

In some embodiments, the 5' end of a nucleic acid strand of the nucleic acid is linked to - O-L¹-.

In some embodiments, both the 3' end and the 5' end of a nucleic acid strand of the nucleic acid are linked to -O-L¹-.

In some embodiments, the nucleic acid is a double-stranded nucleic acid comprising at least one duplex region within which a first strand nucleic acid is at least partially complementary to a second strand nucleic acid, wherein:
(1) neither end of the first strand nucleic acid is linked to -O-L¹-; and the 5' end of the second strand nucleic acid is linked to -O-L¹-, and the 3' end is not linked to -O-L¹-, or the 3' end of the second strand nucleic acid is linked to -O-L¹-, and the 5' end is not linked to -O-L¹-, or both the 3' end and the 5' end of the second strand nucleic acid are linked to -O-L¹-;
(2) neither end of the second strand nucleic acid is linked to -O-L¹-; and the 5' end of the first strand nucleic acid is linked to -O-L¹-, and the 3' end is not linked to -O-L¹-, or the 3' end of the first strand nucleic acid is linked to -O-L¹-, and the 5' end is not linked to - O-L¹-, or both the 3' end and the 5' end of the first strand nucleic acid are linked to -O-L¹-;
(3) both the 5' ends of the first strand nucleic acid and the second strand nucleic acid are linked to -O-L¹-, and neither 3' end is linked to -O-L¹-; or both the 3' ends of the first strand nucleic acid and the second strand nucleic acid are linked to -O-L¹-, and neither 5' end is linked to -O-L¹-; or both the 3' ends and the 5' ends of the first strand nucleic acid and the second strand nucleic acid are linked to -O-L¹-; or both the 3' end of the first strand nucleic acid and the 5' end of the second strand nucleic acid are linked to -O-L¹-, and neither the 5' end of the first strand nucleic acid nor the 3' end of the second strand nucleic acid is linked to -O-L¹-; or both the 5' end of the first strand nucleic acid and the 3' end of the second strand nucleic acid are linked to -O-L¹-, and neither the 3' end of the first strand nucleic acid nor the 5' end of the second strand nucleic acid is linked to -O-L¹-; or
(4) both the 3' end and the 5' end of the first strand nucleic acid are linked to -O-L¹-, and either the 5' end or the 3' end of the second strand nucleic acid is linked to -O-L¹-; or both the 3' end and the 5' end of the second strand nucleic acid are linked to -O-L¹-, and either the 5' end or the 3' end of the first strand nucleic acid is linked to -O-L¹-.

The present disclosure provides a conjugate of the following structure or a pharmaceutically acceptable salt thereof, wherein A is a nucleic acid, and the definition and selection range of the nucleic acid are as described in any one of the embodiments in the context of the present disclosure: 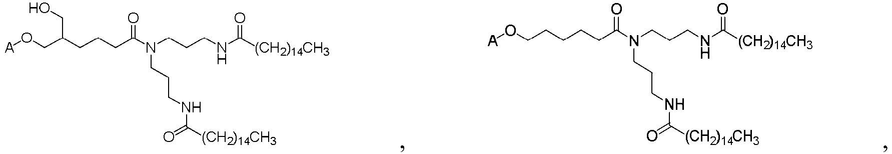 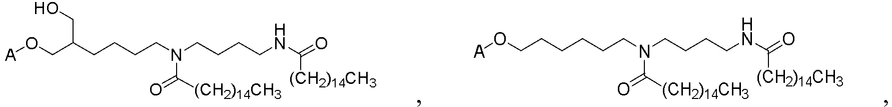 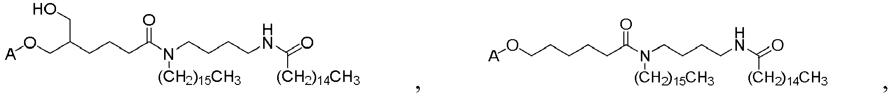 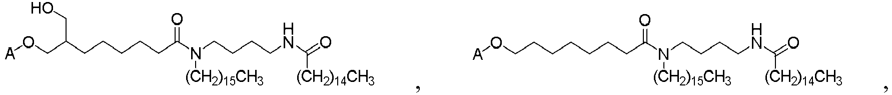 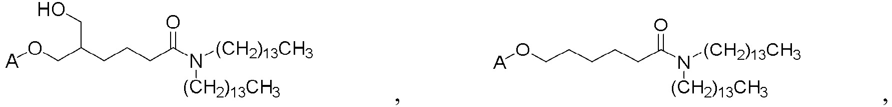 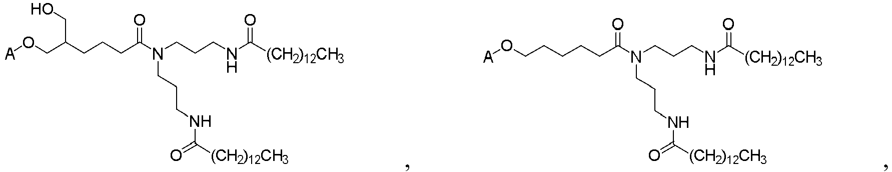 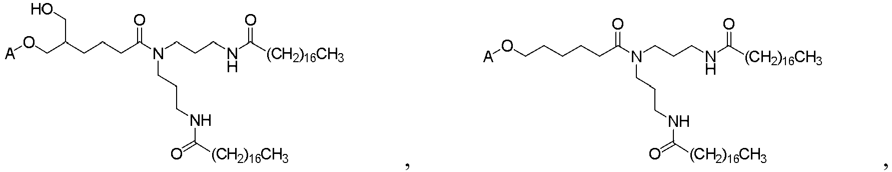 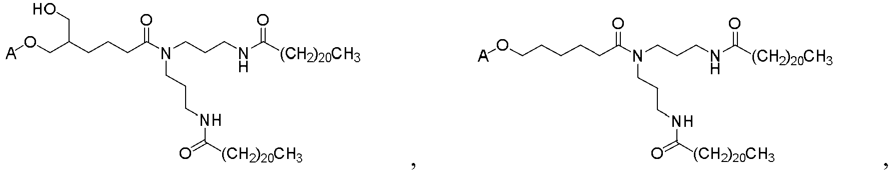 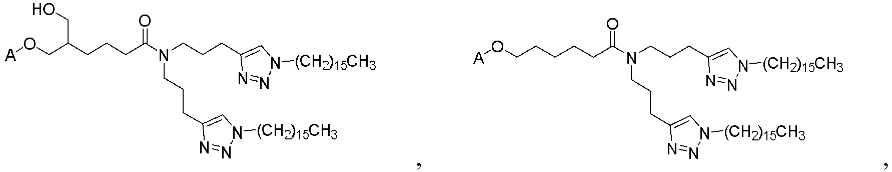 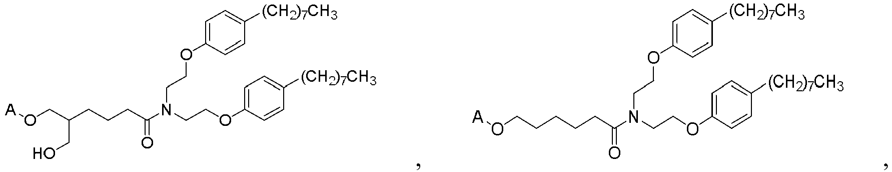 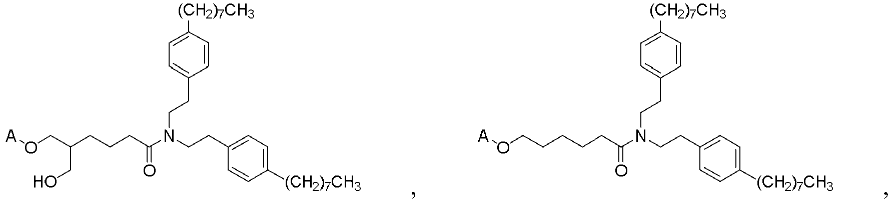 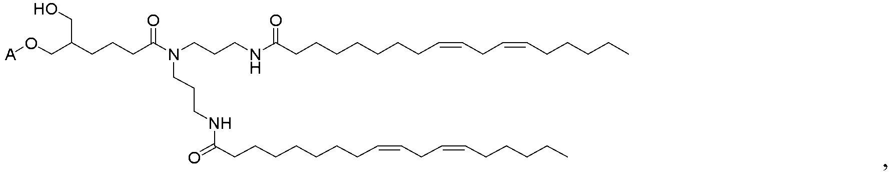 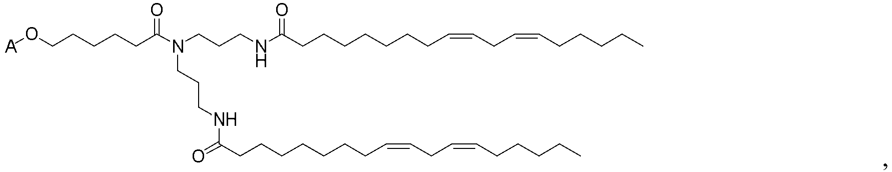 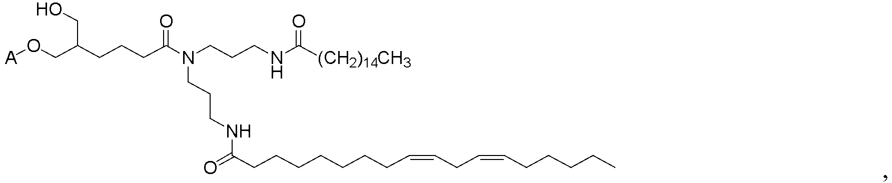 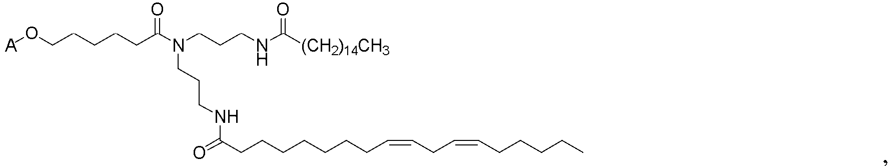 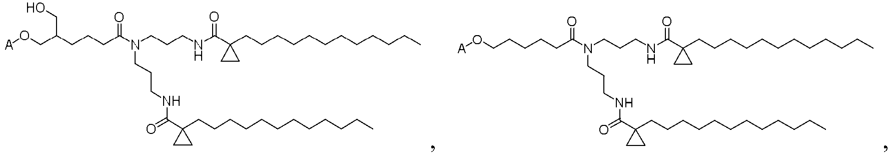 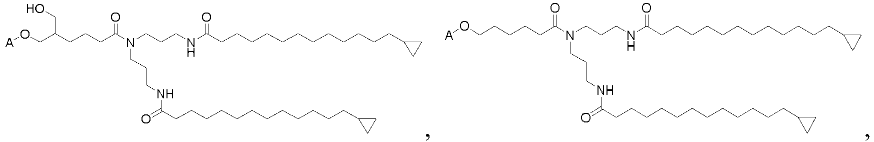 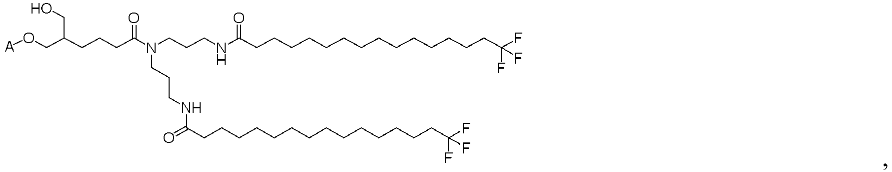 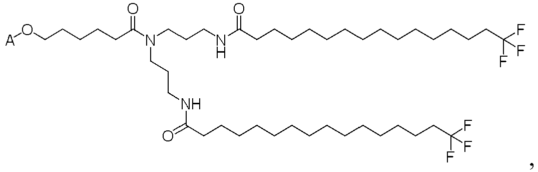 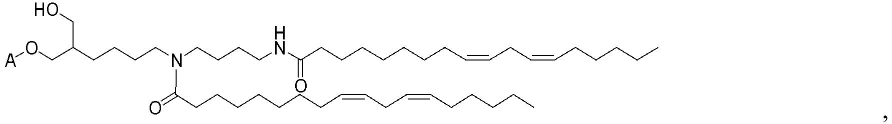 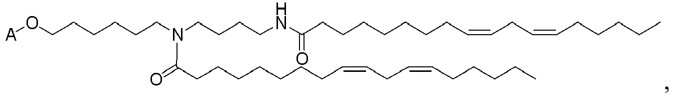 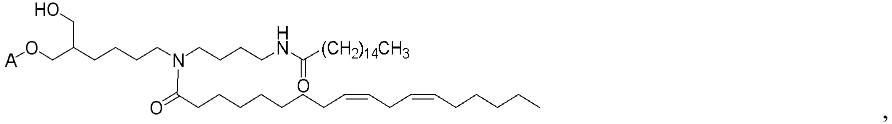 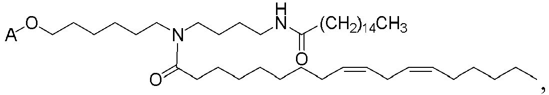 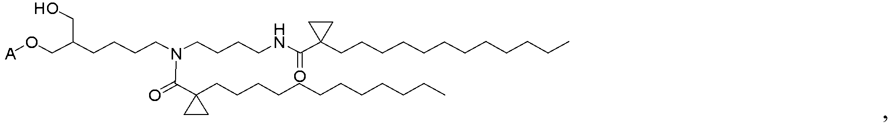 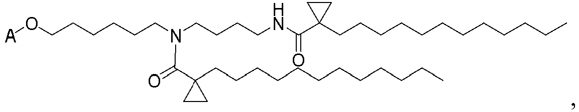 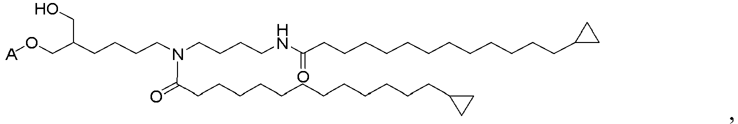 or

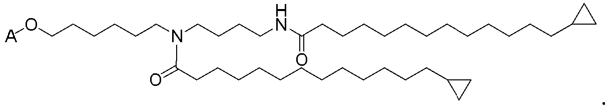

The lipophilic moiety-containing nucleic acid compound represented by formula I of the present disclosure has improved hydrophobicity and increased membrane permeability, and can be effectively delivered into the cytoplasm. The membrane permeability of the oligonucleotide can be improved. Moreover, the oligonucleotide comprising the modification can exert an RNA interference effect in non-hepatic tissues.

The present disclosure provides a compound represented by formula II or a pharmaceutically acceptable salt thereof, 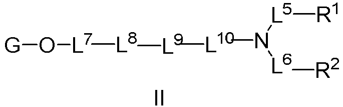
wherein the definitions and selection ranges of L⁵, L⁶, R¹, and R² are described in any one of the embodiments in the context of the present disclosure;
G is hydrogen, 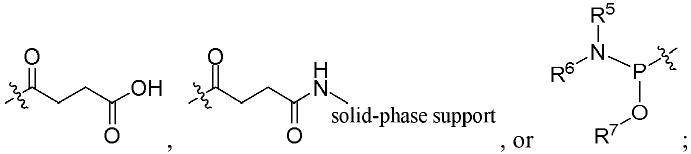
R⁵, R⁶, and R⁷ are each independently C₁₋₆ alkyl unsubstituted or substituted with one or more substituents, and the substituents are halogen, -OH, or -CN;
L⁷, L⁸, L⁹, and L¹⁰ are each independently a bond, -O-, -S-, -NH-, -C(=O)-, -OC(=O)-, - C(=O)O-, -NHC(=O)-, -C(=O)NH-, or unsubstituted or R⁸-substituted C₁₋₁₀ alkyl;
R⁸ is a hydroxy protecting group.

In some embodiments,
G is hydrogen, 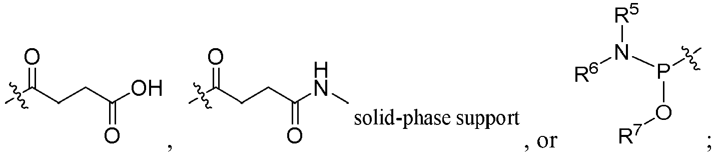
R⁵, R⁶, and R⁷ are each independently C₁₋₆ alkyl unsubstituted or substituted with one or more substituents, and the substituents are halogen, -OH, or -CN;
L⁷, L⁸, L⁹, and L¹⁰ are each independently a bond, -NH-, -C(=O)-, -NHC(=O)-, - C(=O)NH-, or unsubstituted or R⁸-substituted C₁₋₁₀ alkyl;
R⁸ is a hydroxy protecting group;
L⁵ is -L^{5A}-L^{5B}-L^{5C}-L^{5D}-;
L⁶ is -L^{6A}-L^{6B}-L^{6C-}L^{6D}-;
L^{5A}, L^{5B}, L^{5C}, L^{5D}, L^{6A}, L^{6B}, L^{6C}, and L^{6D} are each independently a bond, -O-, -NH-, - C(=O)-, -NHC(=O)-, -C(=O)NH-, -(CH₂)ₘ₁-, unsubstituted 3-10 membered heteroaryl, or unsubstituted 6-14 membered aryl;
R¹ and R² are each independently unsubstituted saturated C₁₋₂₅ hydrocarbyl, unsubstituted unsaturated C₃₋₂₅ hydrocarbyl containing 1-10 carbon-carbon double bonds, or saturated C₁₋₂₅ hydrocarbyl substituted with one or more R¹⁰;
R¹⁰ is halogen or 3-6 membered cycloalkyl;
or when a carbon atom has two R¹⁰ substituents, the two R¹⁰ substituents, together with the carbon atom to which they are attached, form 3-6 membered cycloalkyl;
m1 is independently 1, 2, 3, 4, 5, 6, 7, 8, 9, or 10.

In some embodiments, G is H.

In some embodiments, G is

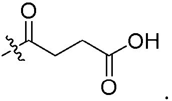

In some embodiments, G is 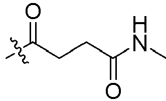 .

In some embodiments, the solid-phase support is a solid-phase support conventionally used in the art, including but not limited to controlled pore glass (CPG), macroporous aminomethyl resin, and polystyrene (PS)-based supports.

In some embodiments, G is 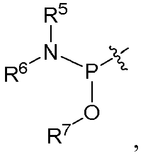 and the definitions and selection ranges of R⁵, R⁶, and R⁷ are as described in any one of the embodiments in the context of the present disclosure.

In some embodiments, in R⁵, R⁶, and R⁷, the number of the substituents is 1, 2, or 3.

In some embodiments, R⁵, R⁶, and R⁷ are each independently C₁₋₄ alkyl unsubstituted or substituted with one or more substituents, and the substituents are halogen, hydroxy, or cyano.

In some embodiments, R⁵, R⁶, and R⁷ are each independently methyl, ethyl, propyl, or isopropyl unsubstituted or substituted with one or more substituents, and the substituents are fluorine, chlorine, bromine, hydroxy, or cyano.

In some embodiments, R⁵ and R⁶ are isopropyl.

In some embodiments, R⁷ is

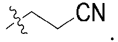

In some embodiments, G is

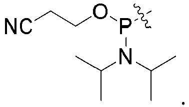

In some embodiments, the hydroxy protecting group is an ester group protecting group, an alkoxymethyl protecting group, an alkyl protecting group, a silyl protecting group, or an aryl protecting group.

In some embodiments, the hydroxy protecting group is an aryl protecting group.

In some embodiments, the hydroxy protecting group is MMTr or DMTr.

In some embodiments, the hydroxy protecting group is DMTr.

In some embodiments, in L⁷, L⁸, L⁹, and L¹⁰, the C₁₋₁₀ alkyl is C₁ alkyl, C₂ alkyl, C₃ alkyl, C₄ alkyl, C₅ alkyl, C₆ alkyl, C₇ alkyl, C₈ alkyl, C₉ alkyl, or C₁₀ alkyl.

In some embodiments, in L⁷, L⁸, L⁹, and L¹⁰, the C₁₋₁₀ alkyl is C₄₋₈ alkyl.

In some embodiments, in L⁷, L⁸, L⁹, and L¹⁰, the C₁₋₁₀ alkyl is C₁₋₄ alkyl.

In some embodiments, in L⁷, L⁸, L⁹, and L¹⁰, the C₁₋₁₀ alkyl is linear or branched C₁₋₁₀ alkyl.

In some embodiments, L⁷, L⁸, L⁹, and L¹⁰ are each independently a bond, -NH-, -C(=O)-, -NHC(=O)-, -C(=O)NH-, or unsubstituted or R⁸-substituted C₁₋₁₀ alkyl, and the definition and selection range of R⁸ are as described in any one of the embodiments in the context of the present disclosure.

In some embodiments, -L⁷-L⁸-L⁹-L¹⁰- is -R⁸-substituted C₁₋₁₀ alkyl-C(=O)- or -R⁸-substituted C₁₋₁₀ alkyl-, and the definition and selection range of R⁸ are as described in any one of the embodiments in the context of the present disclosure. In some embodiments, G is 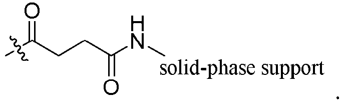 In some embodiments, G is hydrogen.

In some embodiments, -L⁷-L⁸-L⁹-L¹⁰- is -R⁸-substituted C₄₋₈ alkyl-C(=O)- or -R⁸-substituted C₄₋₈ alkyl-, and the definition and selection range of R⁸ are as described in any one of the embodiments in the context of the present disclosure. In some embodiments, G is 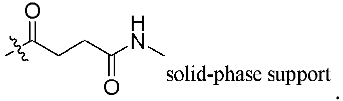 In some embodiments, G is hydrogen.

In some embodiments, -L⁷-L⁸-L⁹-L¹⁰- is -R⁸-substituted C₈ alkyl-NH-C(=O)-, -R⁸-substituted C₅ alkyl-C(=O)-, -R⁸-substituted C₆ alkyl-C(=O)-, -R⁸-substituted C₇ alkyl-C(=O)-, -R⁸-substituted C₈ alkyl-C(=O)-, -R⁸-substituted C₅ alkyl-, -R⁸-substituted C₆ alkyl-, -R⁸-substituted C₇ alkyl-, or -R⁸-substituted C₈ alkyl-, and the definition and selection range of R⁸ are as described in any one of the embodiments in the context of the present disclosure. In some embodiments, G is 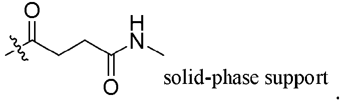 In some embodiments, G is hydrogen.

In some embodiments, L⁷, L⁸, L⁹, and L¹⁰ are each independently a bond, -NH-, -C(=O)-, -NHC(=O)-, -C(=O)NH-, -(CH₂)ₘ₅-, or -(CH₂)ₘ₆-CH(CH₂R⁸)-(CH₂)ₘ₇-, wherein m5, m6, and m7 are each independently 1, 2, 3, 4, 5, 6, 7, 8, 9, or 10, and the definition and selection range of R⁸ are as described in any one of the embodiments in the context of the present disclosure. In some embodiments, G is 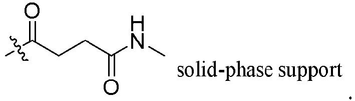 In some embodiments, G is hydrogen.

In some embodiments, -L⁷-L⁸-L⁹-L¹⁰- is -(CH₂)ₘ₆-CH(CH₂R⁸)-(CH₂)ₘ₇-C(=O)- or - (CH₂)ₘ₆-CH(CH₂R⁸)-(CH₂)ₘ₇-, wherein m6 and m7 are each independently 1, 2, 3, 4, 5, 6, 7, 8, 9, or 10, and the definition and selection range of R⁸ are as described in any one of the embodiments in the context of the present disclosure. In some embodiments, G is 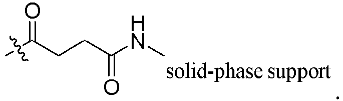 In some embodiments, G is hydrogen.

In some embodiments, -L⁷-L⁸-L⁹-L¹⁰- is 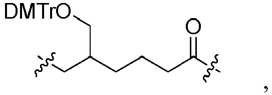 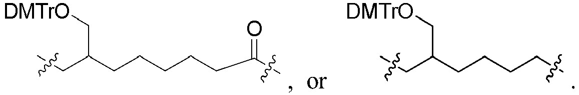 In some embodiments, G is 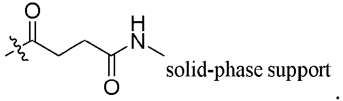 In some embodiments, G is hydrogen.

In some embodiments, the number of R⁸ is 1, 2, or 3.

In some embodiments, -L⁷-L⁸-L⁹-L¹⁰- is -unsubstituted C₁₋₁₀ alkyl-C(=O)- or - unsubstituted C₁₋₁₀ alkyl-. In some embodiments, G is 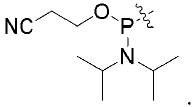 In some embodiments, G is hydrogen.

In some embodiments, -L⁷-L⁸-L⁹-L¹⁰- is -unsubstituted C₄₋₈ alkyl-C(=O)- or - unsubstituted C₄₋₈ alkyl-. In some embodiments, G is 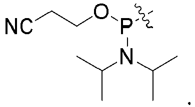 In some embodiments, G is hydrogen.

In some embodiments, -L⁷-L⁸-L⁹-L¹⁰- is -unsubstituted C₅ alkyl-C(=O)-, -unsubstituted C₆ alkyl-C(=O)-, -unsubstituted C₇ alkyl-C(=O)-, -unsubstituted C₈ alkyl-C(=O)-, - unsubstituted C₅ alkyl-, -unsubstituted C₆ alkyl-, -unsubstituted C₇ alkyl-, or - unsubstituted C₈ alkyl-. In some embodiments, G is 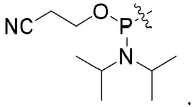 In some embodiments, G is hydrogen.

In some embodiments, -L⁷-L⁸-L⁹-L¹⁰- is -(CH₂)ₘ₅-C(=O)- or -(CH₂)ₘ₅-, wherein m5 is independently 1, 2, 3, 4, 5, 6, 7, 8, 9, or 10. In some embodiments, G is

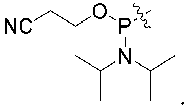

In some embodiments, G is hydrogen.

In some embodiments, -L⁷-L⁸-L⁹-L¹⁰- is 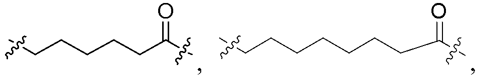 or 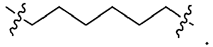 In some embodiments, G is 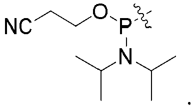 In some embodiments, G is hydrogen.

In some embodiments, the compound represented by formula II is a compound represented by II-1 or II-2: 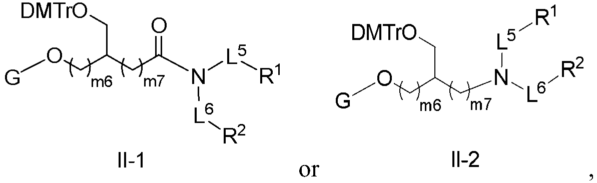 wherein m6 and m7 are each independently 1, 2, 3, 4, 5, 6, 7, 8, 9, or 10, and the definitions and selection ranges of R¹, R², G, L⁵, and L⁶ are as described in any one of the embodiments in the context of the present disclosure. In some embodiments, G is 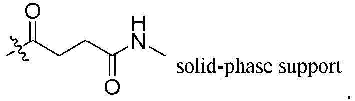 In some embodiments, G is hydrogen.

In some embodiments, the compound represented by formula II is a compound represented by formula II-3, II-4, or II-5: 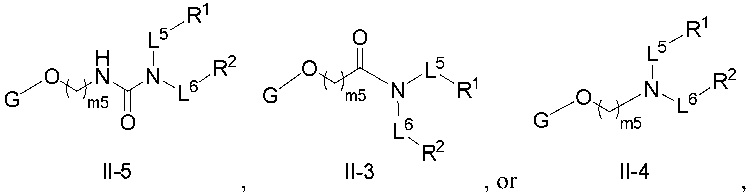 wherein m5 is independently 1, 2, 3, 4, 5, 6, 7, 8, 9, or 10, and the definitions and selection ranges of R¹, R², G, L⁵, and L⁶ are as described in any one of the embodiments in the context of the present disclosure. In some embodiments, G is 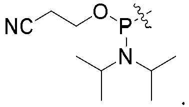 In some embodiments, G is hydrogen.

In some embodiments, the compound represented by formula II is a compound represented by formula II-1A or II-2A: 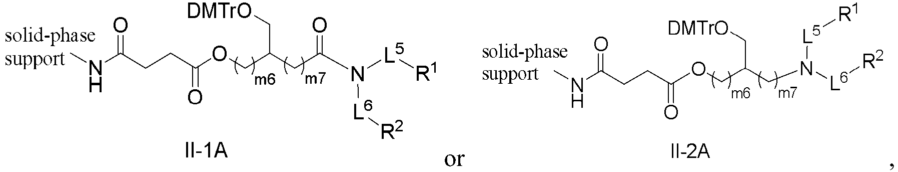 wherein m6 and m7 are each independently 1, 2, 3, 4, 5, 6, 7, 8, 9, or 10, and the definitions and selection ranges of R¹, R², L⁵, and L⁶ are as described in any one of the embodiments in the context of the present disclosure.

In some embodiments, the compound represented by formula II is a compound represented by formula II-3A or II-4A: 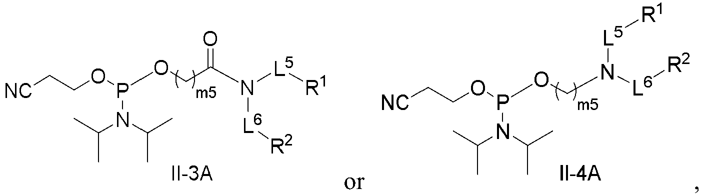 wherein m5 is independently 1, 2, 3, 4, 5, 6, 7, 8, 9, or 10, and the definitions and selection ranges of R¹, R², L⁵, and L⁶ are as described in any one of the embodiments in the context of the present disclosure.

The present disclosure provides a compound of the following structure or a pharmaceutically acceptable salt thereof, wherein 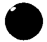 is a solid-phase support, and the solid-phase support is as defined in any one of the embodiments of formula II: 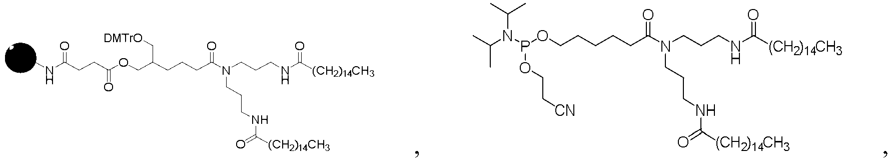 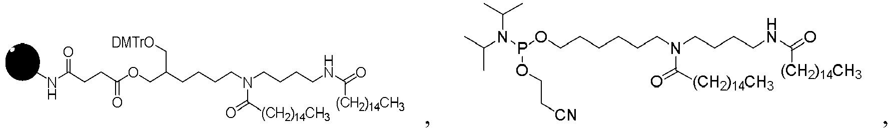 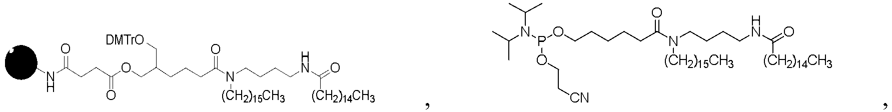 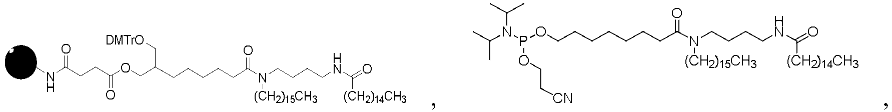 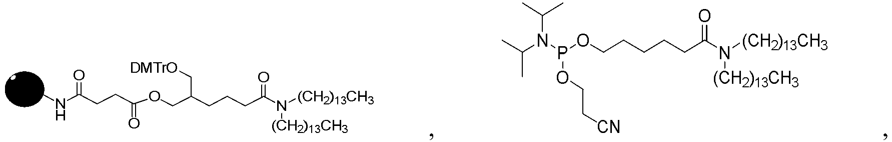 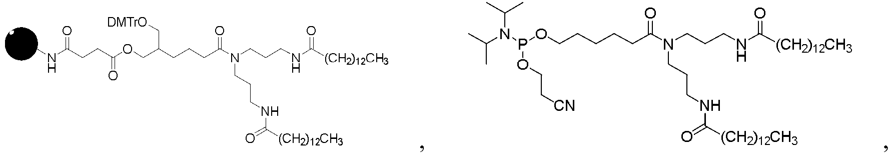 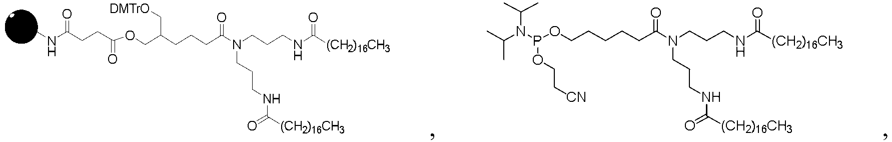 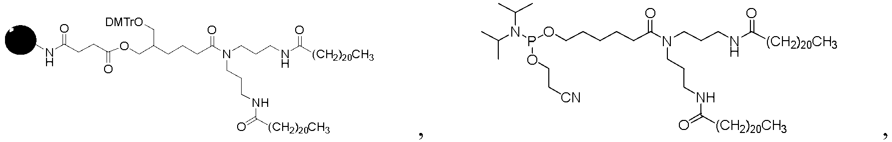 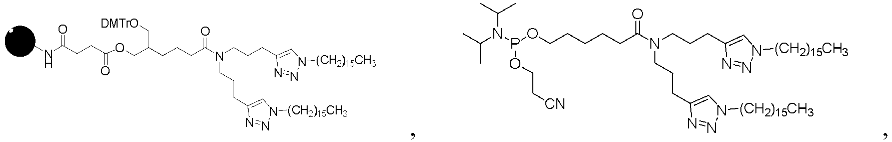 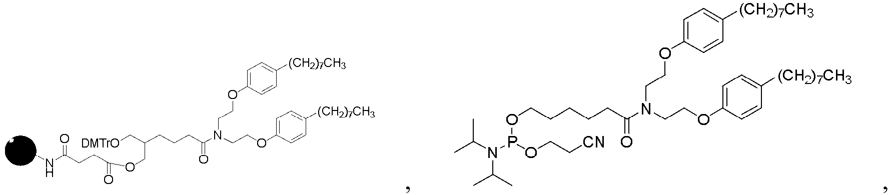 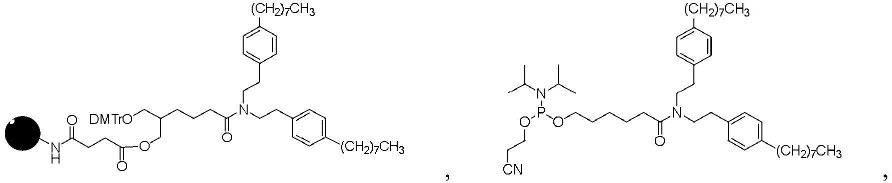 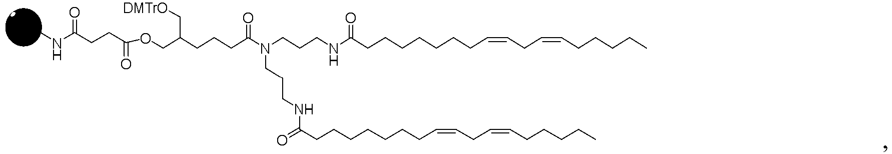 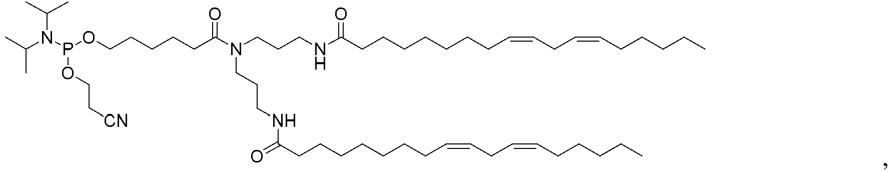 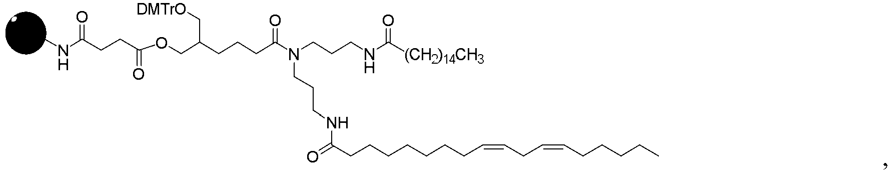 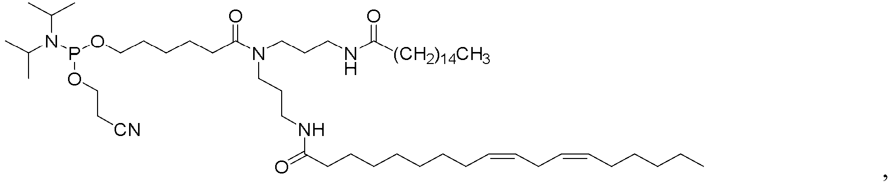 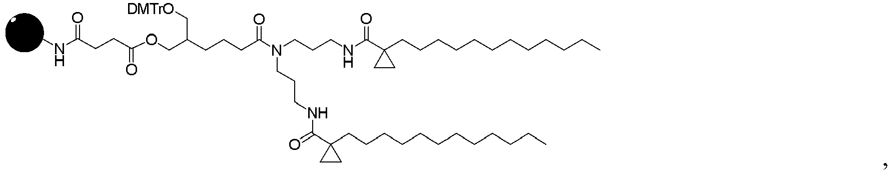 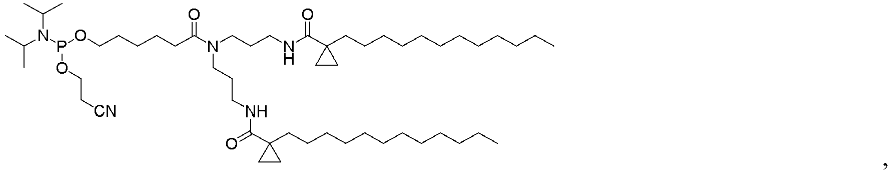 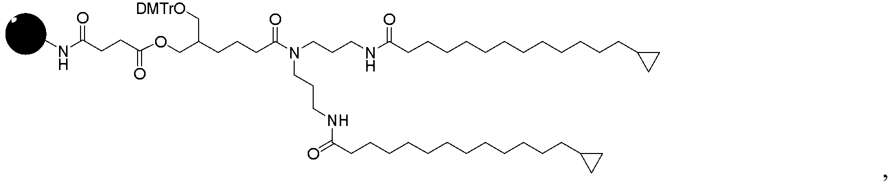 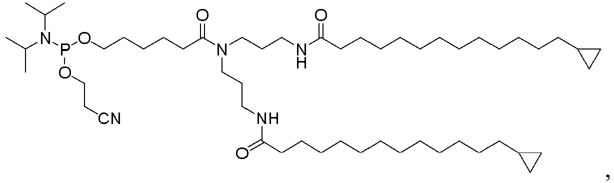 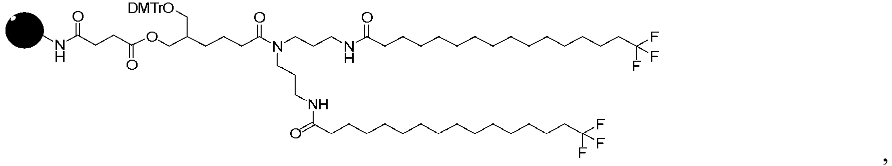 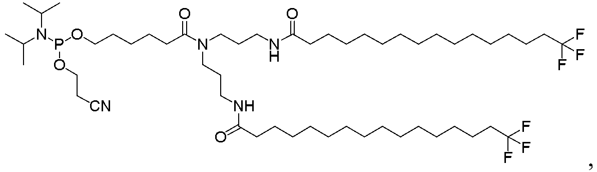 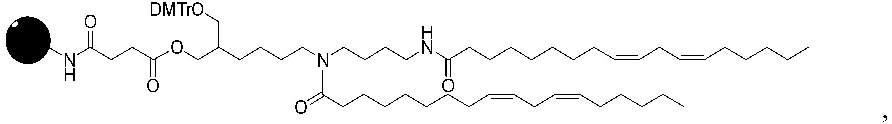 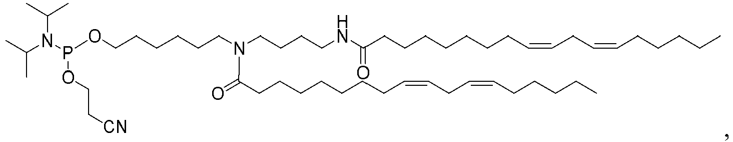 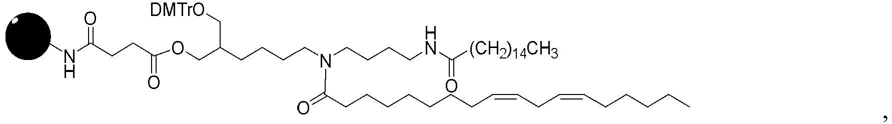 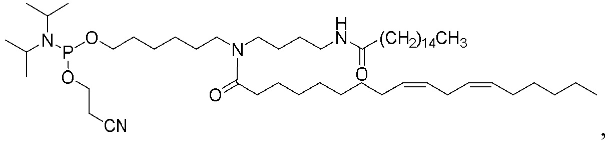 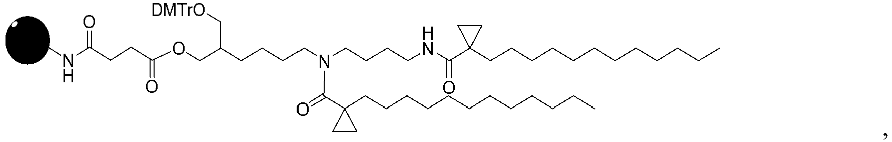 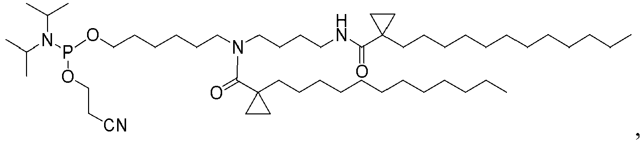 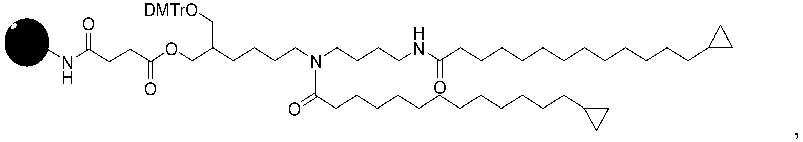 or

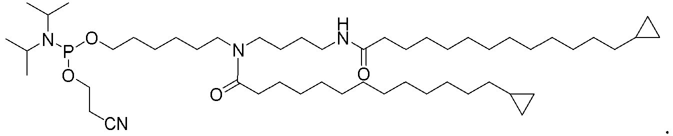

The present disclosure provides an RNAi agent comprising the conjugate or the pharmaceutically acceptable salt thereof described above.

The present disclosure provides a pharmaceutical composition comprising the conjugate or the pharmaceutically acceptable salt thereof described above or the RNAi agent described above, and one or more pharmaceutically acceptable excipients. The term "pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity. In some embodiments, the conjugate or the RNAi agent may be present in a therapeutically effective amount.

In some embodiments, the pharmaceutically acceptable excipients may be excipients commonly used in the art, such as a carrier, a vehicle, a diluent, and/or a delivery polymer. In some embodiments, a unit dose of the pharmaceutical composition may be 0.001 mg-1000 mg.

In some embodiments, the pharmaceutically acceptable excipients may be present in an amount of 0.01-99.99%, or 0.1-99.9%, or 0.5%-99.5%, further 1%-99%, and still further 2%-98%, based on the total weight of the pharmaceutical composition.

In certain embodiments, the pharmaceutical composition comprises 0.01-99.99% of the aforementioned compound or a pharmaceutically acceptable salt thereof or an isotopically substituted form thereof based on the total weight of the pharmaceutical composition. In certain embodiments, the pharmaceutical composition comprises 0.1-99.9% of the aforementioned compound or the pharmaceutically acceptable salt thereof or the isotopically substituted form thereof. In certain embodiments, the pharmaceutical composition comprises 0.5%-99.5% of the aforementioned compound or the pharmaceutically acceptable salt thereof or the isotopically substituted form thereof. In certain embodiments, the pharmaceutical composition comprises 1%-99% of the aforementioned compound or the pharmaceutically acceptable salt thereof or the isotopically substituted form thereof. In certain embodiments, the pharmaceutical composition comprises 2%-98% of the aforementioned compound or the pharmaceutically acceptable salt thereof or the isotopically substituted form thereof.

The pharmaceutical composition may be conveniently presented in a unit dosage form. In general, the pharmaceutical composition may be formulated into any suitable dosage form, such as, but not limited to, injections, tablets, capsules, gels, etc. The pharmaceutical composition includes, but is not limited to, a solution, an emulsion, and a liposome-containing formulation.

The present disclosure provides use of the conjugate or the pharmaceutically acceptable salt thereof, the RNAi agent, or the pharmaceutical composition of the present disclosure in the manufacture of a medicament for preventing and/or treating diseases, including but not limited to diseases or disorders of the brain, eyes, liver, kidneys, heart, adipose tissue, lungs, muscles, or spleen.

The present disclosure provides use of the conjugate or the pharmaceutically acceptable salt thereof, the RNAi agent, or the pharmaceutical composition of the present disclosure in the manufacture of a medicament for preventing and/or treating diseases, including but not limited to neurological diseases or disorders, metabolic diseases or disorders, inflammatory diseases or disorders, or cancer.

The present disclosure provides a method for preventing and/or treating diseases, comprising administering to a patient the conjugate or the pharmaceutically acceptable salt thereof, the RNAi agent, or the pharmaceutical composition of the present disclosure. The present disclosure provides use of the conjugate or the pharmaceutically acceptable salt thereof, the RNAi agent, or the pharmaceutical composition of the present disclosure for preventing and/or treating diseases, comprising administering to a patient the conjugate or the pharmaceutically acceptable salt thereof, the RNAi agent, or the pharmaceutical composition of the present disclosure.

In some embodiments, the diseases include, but are not limited to, diseases or disorders of the brain, eyes, liver, kidneys, heart, adipose tissue, lungs, muscles, or spleen.

In some embodiments, the diseases include, but are not limited to, neurological diseases or disorders, metabolic diseases or disorders, inflammatory diseases or disorders, or cancer.

The present disclosure provides the conjugate or the pharmaceutically acceptable salt thereof described above, the RNAi agent described above, or the pharmaceutical composition described above for use in the prevention and/or treatment of diseases.

In some embodiments, the diseases include, but are not limited to, diseases or disorders of the brain, eyes, liver, kidneys, heart, adipose tissue, lungs, muscles, or spleen.

In some embodiments, the diseases include, but are not limited to, neurological diseases or disorders, metabolic diseases or disorders, inflammatory diseases or disorders, or cancer.

The present disclosure provides a cell comprising the conjugate or the RNAi agent described above.

In some embodiments, the cell is a primary cell. In some embodiments, the cell is an adipocyte, a hepatocyte, a fibroblast, an endothelial cell, a renal cell, a human umbilical vein endothelial cell (HUVEC), an adipocyte, a macrophage, a neuronal cell, a muscle cell, or a differentiated primary human skeletal muscle cell.

In some embodiments, the cell is an immortalized cell. In some embodiments, the cell is an NIH3T3 cell, a differentiated 3T3L1 cell, a RAW264.7 cell, or an SH-SY5Y cell.

In some embodiments, the cell is an adipocyte or a hepatocyte.

The present disclosure provides a method for introducing a nucleic acid into a cell, comprising bringing the cell into contact with the conjugate, the RNAi agent, or the pharmaceutical composition described above.

In some embodiments, the cell is a primary cell. In some embodiments, the cell is an adipocyte, a hepatocyte, a fibroblast, an endothelial cell, a renal cell, a human umbilical vein endothelial cell (HUVEC), an adipocyte, a macrophage, a neuronal cell, a muscle cell, or a differentiated primary human skeletal muscle cell.

In some embodiments, the cell is an immortalized cell. In some embodiments, the cell is an NIH3T3 cell, a differentiated 3T3L1 cell, a RAW264.7 cell, or an SH-SY5Y cell.

In some embodiments, the cell is an adipocyte or a hepatocyte.

The present disclosure provides a preparation method for the conjugate described above, and the preparation method is a method I, a method II, or a method III, wherein:
the method I comprises: taking the compound represented by formula II-1A or II-2A described above as a start and linking nucleoside monomers one by one in the 3'-5' direction in an order in which nucleotides are arranged to give the conjugate;
the method II comprises: taking a universal solid-phase support as a start, linking nucleoside monomers one by one in the 3'-5' direction in an order in which nucleotides are arranged, and then linking the compound represented by formula II-3A or II-4A described above to give the conjugate;
the method III comprises: taking the compound represented by formula II-1A or II-2A described above as a start, linking nucleoside monomers one by one in the 3'-5' direction in an order in which nucleotides are arranged, and then linking the compound represented by formula II-3A or II-4A described above to give the conjugate.

In some embodiments, the linking of each nucleoside monomer involves four reactions: deprotection, coupling, capping, and oxidation or sulfurization.

In some embodiments, the method I may further comprise an annealing step after the linking of nucleoside monomers is completed.

In some embodiments, the methods II and III may further comprise an annealing step after the linking of the compound represented by formula II-3A or II-4A described above is completed.

### Terminology

In another aspect, where the present disclosure does not define a particular configuration, the compounds of the present disclosure may exist in particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which fall within the scope of the present disclosure. Additional asymmetric carbon atoms may be present in substituents such as an alkyl group. All such isomers and mixtures thereof fall within the scope of the present disclosure.

In addition, the compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier.

The compounds of the present disclosure may be asymmetric, for example, having one or more stereoisomers. Unless otherwise specified, all stereoisomers are included, for example, enantiomers and diastereomers. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated, and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

The term "alkyl" refers to a saturated aliphatic hydrocarbon group that is a linear or branched group containing 1 to 30 carbon atoms, preferably an alkyl group containing 1 to 12 carbon atoms, more preferably an alkyl group of 1 to 10 carbon atoms, more preferably an alkyl group of 1 to 6 carbon atoms, and further preferably an alkyl group of 1 to 4 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. Alkyl groups containing 1 to 6 carbon atoms are more preferred, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted; when it is substituted, the substituent may be substituted at any available point of attachment, and the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and a carboxylate group.

The term "alkylene" refers to the remainder of an alkane molecule after 2 hydrogen atoms are removed from the alkane molecule, including linear and branched -ylene groups of 1 to 20 carbon atoms. Non-limiting examples of alkylene groups containing 1 to 6 carbon atoms include methylene (-CH₂-), ethylene (e.g., -CH₂CH₂- or -CH(CH₃)-), propylene (e.g., -CH₂CH₂CH₂- or -CH(CH₂CH₃)-), and butylene (e.g., -CH₂CH₂CH₂CH₂-). Unless otherwise specified, alkylene may be substituted or unsubstituted; when it is substituted, the substituent may be substituted at any available point of attachment, and the substituent is preferably one or more groups independently selected from the group consisting of aryl, heteroaryl, and halogen.

The term "cycloalkyl" or "carbocycle" refers to a saturated or partially unsaturated, monocyclic or polycyclic hydrocarbon substituent, and the cycloalkyl ring contains 3 to 20 carbon atoms, preferably 3 to 7 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc. Polycyclic cycloalkyl groups include spiro-ring, fused-ring, and bridged-ring cycloalkyl groups. Cycloalkyl may be substituted or unsubstituted; when it is substituted, the substituent may be substituted at any available point of attachment, and the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3-to 6-membered heterocycloalkoxy, C₃₋₈ cycloalkenyloxy, and 5- to 6-membered aryl or heteroaryl are optionally substituted with one or more groups selected from the group consisting of halogen, hydroxy, oxo, nitro, and cyano.

The cycloalkyl ring may be fused to an aryl or heteroaryl ring, wherein the ring attached to the parent structure is cycloalkyl; non-limiting examples include indanyl, tetrahydronaphthyl, benzocycloheptyl, etc. Cycloalkyl may be optionally substituted or unsubstituted; when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, hydroxy, oxo, nitro, and cyano.

The term "heterocycloalkyl" or "heterocycle" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen, and S(O)ₘ (where m is an integer from 0 to 2), but a ring moiety of -O-O-, -O-S-, or -S-S- is excluded, and the other ring atoms are carbon atoms. Preferably, it contains 3 to 12 ring atoms, 1-4 of which are heteroatoms; more preferably, it contains 3 to 7 ring atoms. Non-limiting examples of monocyclic heterocycloalkyl groups include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, etc. Polycyclic heterocycloalkyl groups include spiro-ring, fused-ring, and bridged-ring heterocycloalkyl groups. Non-limiting examples of "heterocycloalkyl" groups include: 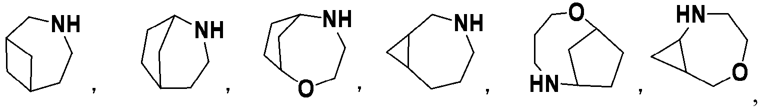 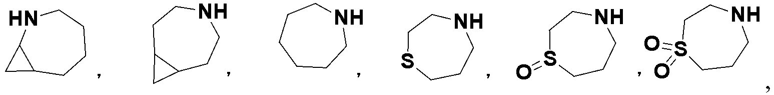 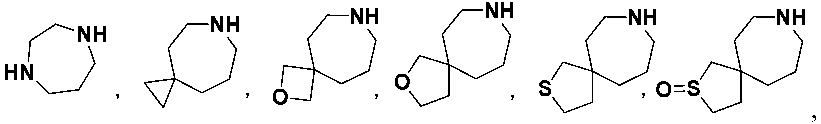 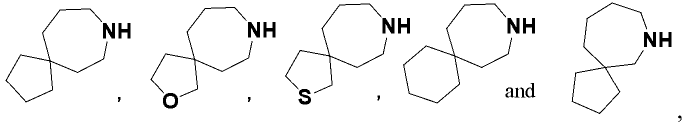 etc.

The heterocycloalkyl ring may be fused to an aryl or heteroaryl ring, wherein the ring attached to the parent structure is heterocycloalkyl; its non-limiting examples include: 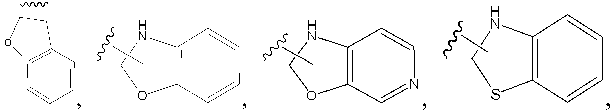 etc.

Heterocycloalkyl may be optionally substituted or unsubstituted; when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, hydroxy, oxo, nitro, and cyano.

The term "aryl" refers to a 6- to 14-membered, preferably 6- to 12-membered, all-carbon monocyclic or fused polycyclic (i.e., rings sharing a pair of adjacent carbon atoms) group having a conjugated π-electron system, such as phenyl and naphthyl. The aryl ring may be fused to a heteroaryl, heterocycloalkyl, or cycloalkyl ring, wherein the ring attached to the parent structure is the aryl ring; its non-limiting examples include:

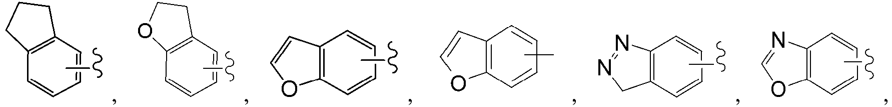

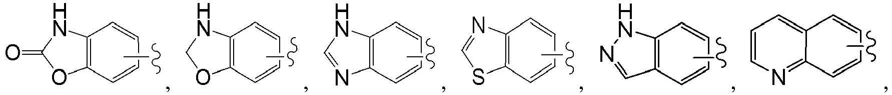

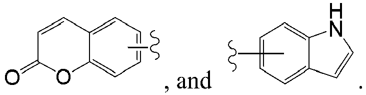

Aryl may be substituted or unsubstituted; when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, hydroxy, oxo, nitro, and cyano.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Heteroaryl is preferably 5- to 12-membered, and is more preferably 5- or 6-membered. For example, its non-limiting examples include imidazolyl, furanyl, thienyl, thiazolyl, pyrazolyl, oxazolyl, isoxazolyl, pyrrolyl, tetrazolyl, pyridyl, pyrimidinyl, thiadiazole, pyrazinyl, triazolyl, indazolyl, benzimidazolyl, 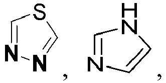 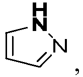 etc.

The heteroaryl ring may be fused to an aryl, heterocycloalkyl, or cycloalkyl ring, wherein the ring attached to the parent structure is the heteroaryl ring; its non-limiting examples include:

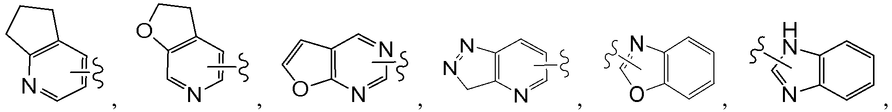

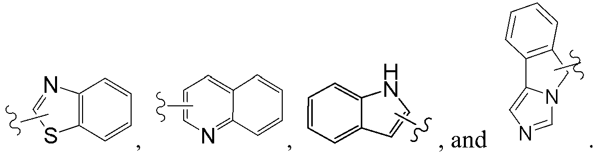

Heteroaryl may be optionally substituted or unsubstituted; when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, hydroxy, oxo, nitro, and cyano.

The term "spiro-ring" refers to compounds in which two rings share one atom.

The term "spirocycloalkyl" refers to a 5- to 20-membered polycyclic group in which one carbon atom (referred to as a spiro atom) is shared between monocyclic rings; it may contain one or more double bonds, but no ring has a fully conjugated π-electron system. It is preferably 6- to 14-membered, and is more preferably 7- to 10-membered. According to the number of spiro atoms shared between rings, spirocycloalkyl may be monospirocycloalkyl, bispirocycloalkyl, or polyspirocycloalkyl, preferably monospirocycloalkyl and bispirocycloalkyl, and more preferably 4-membered/4-membered, 4-membered/5-membered, 4-membered/6-membered, 5-membered/5-membered, or 5-membered/6-membered monospirocycloalkyl. "Spirocarbocycle" refers to the ring system in spirocycloalkyl. Non-limiting examples of spirocycloalkyl groups include:

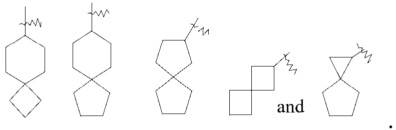

The term "spiroheterocyclyl" refers to a 5- to 20-membered polycyclic heterocyclyl group in which one atom (referred to as a spiro atom) is shared between monocyclic rings, wherein one or more ring atoms are heteroatoms selected from the group consisting of nitrogen, oxygen, and S(O)ₘ (where m is an integer from 0 to 2), and the other ring atoms are carbon atoms. It may contain one or more double bonds, but no ring has a fully conjugated π-electron system. It is preferably 6- to 14-membered, and is more preferably 7- to 10-membered. According to the number of spiro atoms shared between rings, spiroheterocyclyl may be monospiroheterocyclyl, bispiroheterocyclyl, or polyspiroheterocyclyl, preferably, monospiroheterocyclyl and bispiroheterocyclyl, and more preferably 4-membered/4-membered, 4-membered/5-membered, 4-membered/6-membered, 5-membered/5-membered, or 5-membered/6-membered monospiroheterocyclyl. "Spiroheterocycle" refers to the ring system in spiroheterocyclyl. Non-limiting examples of spiroheterocyclyl groups include:

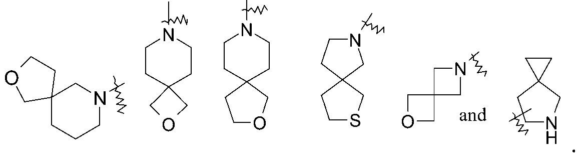

The term "fused-ring" refers to compounds formed by fusing two or more rings by sharing two adjacent atoms.

The term "fused cycloalkyl" refers to a 5- to 20-membered all-carbon polycyclic group in which each ring in the system shares a pair of adjacent carbon atoms with other rings in the system, wherein one or more rings may contain one or more double bonds, but no ring has a fully conjugated π-electron system. It is preferably 6- to 14-membered, and is more preferably 7- to 10-membered. According to the number of constituent rings, it may be bicyclic, tricyclic, tetracyclic, or polycyclic fused cycloalkyl, preferably bicyclic or tricyclic, and more preferably 5-membered/5-membered or 5-membered/6-membered bicycloalkyl. Non-limiting examples of fused cycloalkyl groups include:

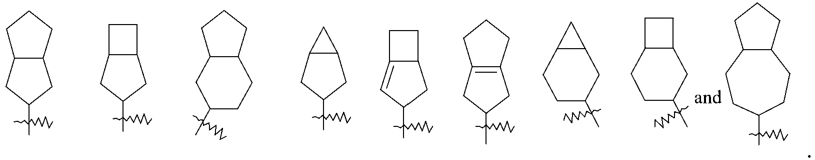

The term "fused heterocyclyl" refers to a 5- to 20-membered polycyclic heterocyclyl group in which each ring in the system shares a pair of adjacent atoms with other rings in the system, wherein one or more rings may contain one or more double bonds, but no ring has a fully conjugated π-electron system; one or more ring atoms are heteroatoms selected from the group consisting of nitrogen, oxygen, and S(O)ₘ (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic fused heterocyclyl, preferably bicyclic or tricyclic, and more preferably 5-membered/5-membered or 5-membered/6-membered bicyclic fused heterocyclyl. "Fused heterocycle" refers to the ring system in fused heterocyclyl. Non-limiting examples of fused heterocyclyl groups include:

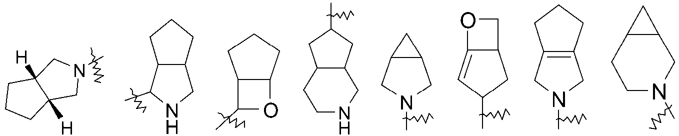

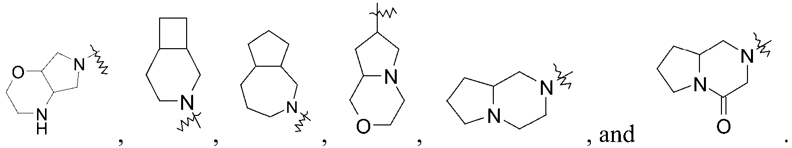

The term "fused heteroaryl" may be an unsaturated aromatic fused ring structure containing 5-14 ring atoms (including at least one heteroatom) formed by connecting two or more cyclic structures that share two adjacent atoms, including the case in which a carbon atom, a nitrogen atom, and a sulfur atom may be substituted with oxo, preferably "5-12 membered fused heteroaryl", "7-12 membered fused heteroaryl", "9-12 membered fused heteroaryl", etc., for example, benzofuranyl, benzoisofuranyl, benzothienyl, indolyl, isoindolyl, benzoxazolyl, benzimidazolyl, indazolyl, benzotriazolyl, quinolyl, 2-quinolinonyl, 4-quinolinonyl, 1-isoquinolinonyl, isoquinolinyl, acridinyl, phenanthridinyl, benzopyridazinyl, phthalazinyl, quinazolinyl, quinoxalinyl, phenazinyl, pteridinyl, purinyl, naphthyridinyl, phenazinyl, and phenothiazinyl. "Fused heteroaromatic ring" refers to the ring system in fused heteroaryl.

Fused heteroaryl may be optionally substituted or unsubstituted; when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and a carboxylate group.

The term "bridged-ring" refers to a structure formed by two or more cyclic structures sharing two non-adjacent ring atoms.

The term "bridged cycloalkyl" refers to a 5- to 20-membered all-carbon polycyclic group in which any two rings share two carbon atoms that are not directly connected; it may contain one or more double bonds, but no ring has a fully conjugated π-electron system. It is preferably 6- to 14-membered, and is more preferably 7- to 10-membered. According to the number of constituent rings, it may be bicyclic, tricyclic, tetracyclic, or polycyclic bridged cycloalkyl, preferably bicyclic, tricyclic, or tetracyclic, and more preferably bicyclic or tricyclic. Non-limiting examples of bridged cycloalkyl groups include:

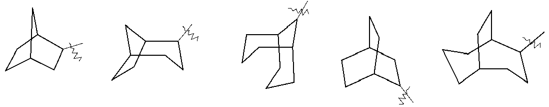

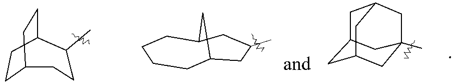

The term "bridged heterocyclyl" refers to a 5- to 14-membered polycyclic heterocyclyl group in which any two rings share two atoms that are not directly connected; it may contain one or more double bonds, but no ring has a fully conjugated π-electron system, wherein one or more ring atoms are heteroatoms selected from the group consisting of nitrogen, oxygen, and S(O)ₘ (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic bridged heterocyclyl, preferably bicyclic, tricyclic, or tetracyclic, and more preferably bicyclic or tricyclic. Non-limiting examples of bridged heterocyclyl groups include:

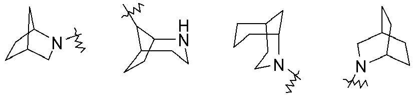

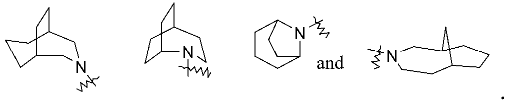

The term "alkoxy" refers to -O-(alkyl) and -O-(unsubstituted cycloalkyl), wherein the alkyl is as defined above. Non-limiting examples of alkoxy groups include methoxy, ethoxy, propoxy, butoxy, cyclopropyloxy, cyclobutoxy, cyclopentyloxy, and cyclohexyloxy. Alkoxy may be optionally substituted or unsubstituted; when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, and 5- to 6-membered aryl or heteroaryl are optionally substituted with one or more groups selected from the group consisting of halogen, hydroxy, oxo, nitro, and cyano. Likewise, the definitions of "alkynyloxy", "alkenyloxy", "cycloalkoxy", "heterocycloalkoxy", and "cycloalkenyloxy" are similar to the above definition of "alkoxy".

The term "hydroxy" refers to -OH.

The term "halogen" refers to fluorine, chlorine, bromine, or iodine.

The term "haloalkyl" refers to an alkyl group substituted with halogen, wherein the alkyl group is as defined above.

The term "cyano" refers to -CN.

The term "nitro" refers to -NO₂.

The term "oxo" refers to the =O group. For example, a carbon atom and an oxygen atom are connected by a double bond to form a ketone or aldehyde group.

The term "amino" refers to -NH₂.

The term "carboxyl" refers to -C(O)OH.

The term "aldehyde group" refers to -CHO.

The term "benzoyl" refers to -Bz.

The term "methoxyphenyldiphenylmethyl" refers to -MMTr.

The term "dimethoxytrityl" refers to -DMTr.

"Optionally" or "optional" means that the event or circumstance subsequently described may, but does not necessarily, occur; this description includes an instance where the event or circumstance occurs or does not occur. For example, "C₁₋₆ alkyl optionally substituted with halogen or cyano" means that the halogen or cyano may, but does not necessarily, exist; this description includes an instance where the alkyl is substituted with halogen or cyano and an instance where the alkyl is not substituted with halogen or cyano.

The term "substituted" means that one or more, preferably up to 5, and more preferably 1 to 3, hydrogen atoms in the group are independently substituted with a corresponding number of substituents. It goes without saying that a substituent is only in its possible chemical position, and those skilled in the art can determine (experimentally or theoretically) possible or impossible substitution without undue effort.

"Substituted with one or more..." refers to substitution with a single substituent or multiple substituents. In the case of substitution with multiple substituents, there may be a plurality of identical substituents, or one or a group of a plurality of different substituents. The term "link", when used to refer to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly linked" means that a first compound or group is linked to a second compound or group without any atom or group of atoms interposed between. The term "indirectly linked" means that a first compound or group is linked to a second compound or group by an intermediate group, a compound, or a molecule (e.g., a linking group).

In the chemical structures of the compounds of the present disclosure, the bond " 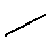 " indicates an unspecified configuration; that is, if chiral isomers exist in the chemical structures, the bond " 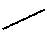 " may be " 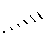 " or " 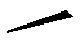 ", or includes both the configurations " 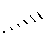 " and " 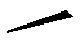 " simultaneously. Although all of the structural formulas described above are drawn as certain isomeric forms for the sake of simplicity, the present disclosure may include all isomers, such as tautomers, rotamers, geometric isomers, diastereomers, racemates, and enantiomers. In the chemical structures of the compounds of the present disclosure, the bond " 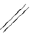 " does not specify a configuration; that is, the configuration of the bond " 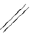 " may be an E configuration or a Z configuration, or includes both the E configuration and the Z configuration simultaneously.

Unless otherwise indicated, the symbol 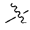 as used herein means that it may be linked to any group or groups according to the scope of the disclosure described herein.

Unless otherwise indicated, the variable may be oriented such that any one point of attachment on the variable is linked to any one point of attachment on the compound of formula I. For example, the variable L₁ has two points of attachment to the compound of formula I. Although L₁ is shown as -(CH₂)₆-O-CH₂- in one embodiment, this embodiment should also be understood as a compound in which L₁ is -CH₂-O-(CH₂)₆-.

In the present disclosure, the terms "comprise" and "include" can be replaced with "consist of".

The term "pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity. The term "pharmaceutically acceptable excipient" includes, but is not limited to, any auxiliary, carrier, excipient, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration as acceptable for use in humans or livestock animals.

Unless otherwise specified, the "compound", "conjugate", and "nucleic acid" of the present disclosure can all independently be present in the form of a salt or mixed salt or in a non-salt form (e.g., a free acid or free base). When present in the form of a salt or mixed salt, it may be a pharmaceutically acceptable salt.

The term "pharmaceutically acceptable salt" includes pharmaceutically acceptable acid addition salts and pharmaceutically acceptable base addition salts.

"Pharmaceutically acceptable acid addition salt" refers to salts formed with inorganic or organic acids, capable of retaining the biological effectiveness of free bases without having any other side effect. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts formed with inorganic bases or organic bases, capable of retaining the biological effectiveness of free acids without having any other side effect. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts, and magnesium salts; sodium salts are preferred. Salts derived from organic bases include, but are not limited to, salts of the following: primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

As used herein, the term "inhibit" is used interchangeably with "decrease", "silence", "down-regulate", "repress", and other similar terms, and includes any level of inhibition. Inhibition can be assessed in terms of a decrease in the absolute or relative level of one or more of these variables relative to a control level. The control level may be any type of control level used in the art, such as a pre-dose baseline level or a level determined from a similar untreated or control (e.g., buffer-only control or inert agent control) treated subject, cell, or sample. For example, the remaining mRNA expression level can be used to characterize the degree of inhibition of target gene expression by siRNA; for example, the remaining mRNA expression level is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition rate of target gene expression can be measured using a Dual-Glo^{®} Luciferase Assay System: the Firefly chemiluminescence value and the Renilla chemiluminescence value are read, and the relative value Ratio = Ren/Fir is calculated. In the present disclosure, the ratio of the remaining mRNA expression level (or residual activity%) = Ratio (siRNA-treated group)/Ratio (siRNA-free control group), and the inhibition rate (%) = 100% - the remaining mRNA expression level (%).

"Effective amount", "effective dose", "effective therapeutic amount", or "therapeutically effective amount" refers to the amount of a drug, a compound, or a pharmaceutical composition necessary to obtain any one or more beneficial or desired therapeutic results. For prophylactic use, the beneficial or desired results include elimination or reduction of risk, reduction of severity, or delay of the onset of a disorder, including the biochemistry, histology, and/or behavioral symptoms of the disorder, complications thereof, and intermediate pathological phenotypes that appear during the progression of the disorder. For therapeutic applications, the beneficial or desired results include clinical results, such as reducing the incidence of various disorders related to the target gene, target mRNA, or target protein of the present disclosure or ameliorating one or more symptoms of the disorders, reducing the dose of other agents required to treat the disorders, enhancing the therapeutic effect of another agent, and/or delaying the progression of disorders related to the target gene, target mRNA, or target protein of the present disclosure in patients. An effective amount also means an amount sufficient to allow or facilitate diagnosis. The effective amount for a particular patient or veterinary subject may vary depending on factors such as the disorder to be treated, the general health of the patient, the method and route and dose of administration, and the severity of side effects. An effective amount may be the maximum dose or administration regimen to avoid significant side effects or toxic effects.

As used herein, "object", "patient", "subject", and "individual" are used interchangeably and include human or non-human animals, e.g., mammals, e.g., humans or monkeys.

In the present disclosure, "phosphoester group" and "phosphate linkage" are used interchangeably and include phosphomonoesters, phosphodiesters, or phosphotriesters. The "phosphoester group" in the "phosphorothioate group" also has the same meaning. Unless otherwise specified, the natural internucleotide phosphoester group is a phosphodiester group.

As used herein, in the context of RNA-mediated gene silencing, the sense strand (also referred to as SS or SS strand) of an siRNA refers to a strand that comprises a sequence that is identical or substantially identical to a target mRNA sequence; the antisense strand (also referred to as AS or AS strand) of an siRNA refers to a strand having a sequence complementary to a target mRNA sequence.

As used herein, the first strand may be referred to as the antisense strand, and the second strand may be referred to as the sense strand. The terms first strand and antisense strand or second strand and sense strand should be considered interchangeable.

In the present disclosure, the "5' region", "5' end", and "5' terminus" of the sense or antisense strand are used interchangeably. For example, the nucleotides at positions 2 to 8 of the 5' region of the antisense strand may also be replaced with the nucleotides at positions 2 to 8 of the 5' end of the antisense strand. Likewise, the "3' region", "3' terminus", and "3' end" of the sense or antisense strand are also used interchangeably.

As used herein, the term "base" encompasses any known DNA and RNA bases and base analogs such as purines or pyrimidines, and also includes the natural compounds adenine, thymine, guanine, cytosine, uracil, inosine, and natural analogs.

Unless otherwise stated, in the context of the present disclosure, the uppercase letters C, G, U, A, and T represent base components of nucleotides; the lowercase letter d indicates that the adjacent nucleotide one the right side of the letter d is a deoxyribonucleotide; the lowercase letter m indicates that the adjacent nucleotide on the left side of the letter m is a methoxy-modified nucleotide; the lowercase letter f indicates that the adjacent nucleotide on the left side of the letter f is a fluoro-modified nucleotide; and the lowercase letter s indicates that the two adjacent nucleotides flanking the letter s are linked by a phosphorothioate group.

As used herein, the terms "complementary" and "reverse complementary" are used interchangeably and have the meaning well-known to those skilled in the art; that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (G) is always paired with the pyrimidine base cytosine (C). Each base pair comprises a purine and a pyrimidine. When adenines of one strand are always paired with thymines (or uracils) of another strand, and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

As used herein, "chemical modification" or "modification" means a structure that has chemical differences when compared with a naturally occurring counterpart, including all changes made by chemical means, such as addition or removal of chemical moieties, or substitution of one chemical moiety for another.

As used herein, the term "fluoro-modified nucleotide" refers to a nucleotide formed by substituting the hydroxy group at the 2' position of the ribosyl group of the nucleotide with fluorine; the methoxy-modified nucleotide refers to a nucleotide formed by substituting the 2'-hydroxy group of the ribosyl group with a methoxy group.

As used herein, the term "nucleic acid" includes, but is not limited to, oligonucleotides, single-stranded oligonucleotides, single-stranded antisense oligonucleotides, short interfering RNAs (siRNAs), double-stranded RNAs (dsRNAs), microRNAs (miRNAs), short hairpin RNAs (shRNAs), ribozymes, interfering RNA molecules, and Dicer enzyme substrates. As used herein, the term "RNAi agent" refers to an agent that contains an RNA or RNA-like (e.g., chemically modified RNA) oligonucleotide molecule that is capable of degrading or inhibiting transcription and translation of a target messenger RNA (mRNA) in a sequence-specific manner. In the present disclosure, RNAi agents may operate through the RNA interference mechanism (i.e., inducing RNA interference through interaction with the RNA interference pathway machinery (RNA-induced silencing complex or RISC) of mammalian cells), or act by any other mechanisms or pathways.

The RNAi agents described herein include nucleic acids.

### BRIEF DESCRIPTION OF THE DRAWINGS

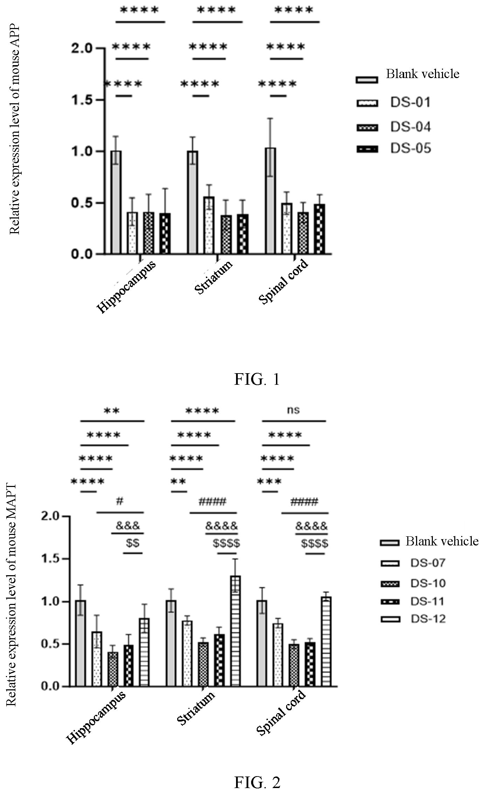

FIG. 1 shows the mouse APP mRNA expression level inhibitory activity of DS-01, DS-04, and DS-05, wherein the data results are expressed as mean ± SD. In the drawing, **** represents *p* < 0.0001 compared to the blank vehicle.
FIG. 2 shows the mouse MAPT mRNA expression level inhibitory activity of DS-07, DS-10, DS-11, and DS-12, wherein the data results are expressed as mean ± SD. In the drawing, **** represents *p* < 0.0001 compared to the blank vehicle, *** represents *p* < 0.001 compared to the blank vehicle, ** represents *p* < 0.01 compared to the blank vehicle, ns means that there was no significance compared to the blank vehicle, #### represents *p* < 0.0001 compared to DS-12, # represents *p* < 0.05 compared to DS-12, &&& represents *p* < 0.001 compared to DS-12, &&&& represents *p* < 0.0001 compared to DS-12, $$ represents *p* < 0.01 compared to DS-12, and $$$$ represents *p* < 0.0001 compared to DS-12.

### DETAILED DESCRIPTION

The present disclosure is further described below with reference to examples; however, these examples are not intended to limit the scope of the present disclosure. Experimental methods without specific conditions indicated in the examples of the present disclosure were generally conducted under conventional conditions, or conditions recommended by the manufacturers of the starting materials or commercial products. Where the specific source of a reagent is not indicated, the reagent may be obtained from any supplier of molecular biology reagents at a quality/purity level for molecular biology applications. Unless otherwise specified, all reagents used in the following examples were commercially available.

### Example 1: Synthesis of Compound A01

The synthesis scheme for compound **A01** is as follows:

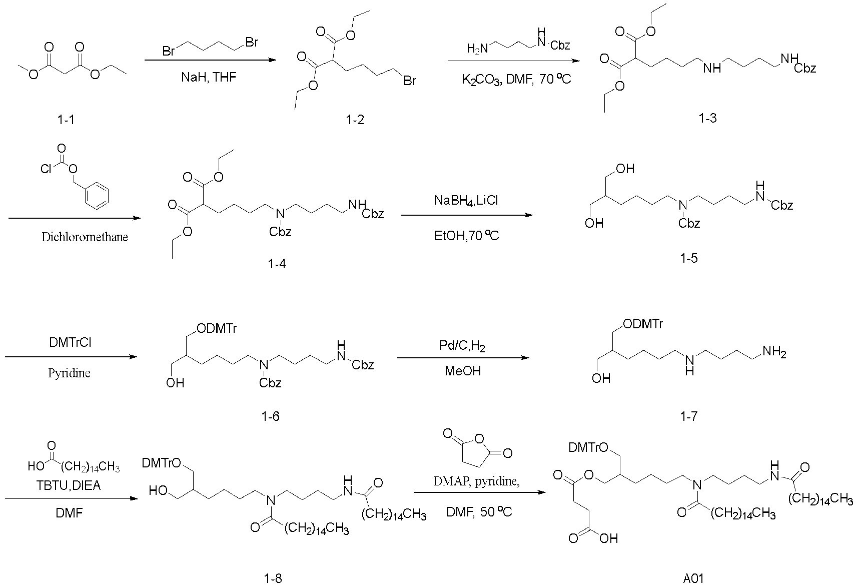

The synthesis and identification of specific intermediates and final products involved in the above scheme are as follows:

### Compound 1-2:

Compound **1-1** (10.0 g, 62.4 mmol) and 1,4-dibromobutane (47.18 g, 218.5 mmol) were added to dimethyl sulfoxide (30 mL). In a nitrogen atmosphere, the mixture was cooled to below 10 °C, and sodium hydride (2.5 g, 62.4 mmol) was added in portions. The mixture was left to react for 2 h with the temperature maintained. After LCMS analysis showed that the reaction was complete, water (100 mL) was slowly added to quench the reaction, and extraction was performed with ethyl acetate (50 mL × 2). The organic phases were combined, then washed with saturated brine, dried over anhydrous sodium sulfate, and concentrated, and the resulting crude product was purified and separated by normal-phase silica gel column chromatography to give compound 1-2 (9.50 g, yield: 51%). LCMS: MS (ESI) m/z = 296.2 [M+H]⁺.

### Compound 1-3:

N-Benzyloxycarbonyl-1,4-diaminobutane hydrochloride (4.38 g, 16.9 mmol) and potassium carbonate (3.28 g, 23.7 mmol) were added to N,N-dimethylformamide (20 mL). The mixture was heated to 70 °C and stirred for 30 min, and compound 1-2 (2.0 g, 6.8 mmol) was then added dropwise to the reaction mixture. After the addition, the mixture was stirred for 30 min with the temperature maintained. After LCMS analysis showed that the reaction was complete, the reaction mixture was cooled to room temperature, then poured into water (80 mL) to quench the reaction, and extracted with ethyl acetate (50 mL × 2). The organic phases were combined, then washed with saturated brine, dried over anhydrous sodium sulfate, and concentrated to give a crude product of compound 1-3 (2.0 g, yield: 67%). LCMS: MS (ESI) m/z = 437.6 [M+H]⁺.

### Compound 1-4:

Compound **1-3** (2.0 g, 4.6 mmol) was dissolved in dichloromethane (20 mL), and benzyl chloroformate (0.86 g, 5.0 mmol) was slowly added dropwise at room temperature. After the mixture was left to react at room temperature for 10 min, LCMS analysis showed that the reaction was complete. The reaction mixture was concentrated, and the crude product was purified and separated by normal-phase silica gel column chromatography to give compound **1-4** (0.52 g, yield: 20%). LCMS: MS (ESI) m/z = 571.8 [M+H]⁺.

### Compound 1-5:

Compound **1-4** (1.19 g, 2.1 mmol) was dissolved in ethanol (10 mL). In a nitrogen atmosphere, sodium borohydride (0.29 g, 8.4 mmol) and lithium chloride (0.71 g, 42.4 mmol) were added, and the mixture was stirred at 70 °C for 5 h. After LCMS analysis showed that the reaction was complete, the reaction was cooled to room temperature and quenched by slowly adding a saturated aqueous ammonium chloride solution (30 mL) dropwise, and extraction was performed with ethyl acetate (50 mL × 2). The organic phases were combined, then washed with saturated brine, dried over anhydrous sodium sulfate, and concentrated, and the resulting crude product was purified and separated by normal-phase silica gel column chromatography to give compound **1-5** (0.80 g, yield: 78%). LCMS:MS (ESI) m/z = 486.6 [M+Na]⁺.

### Compound 1-6:

Compound **1-5** (1.40 g, 2.9 mmol) was dissolved in a solution of pyridine (14 mL), and several molecular sieve beads were added. The mixture was stirred at room temperature for 10 min, and 4,4'-dimethoxytrityl chloride (1.07 g, 3.2 mmol) was then added. The mixture was left to react at room temperature for 5 h. After LCMS analysis showed that the reaction was complete, the reaction mixture was concentrated, and the resulting crude product was separated by reversed-phase column chromatography (acetonitrile/water) to give compound **1-6** (1.50 g, yield: 66%). LCMS:MS (ESI) m/z = 812.1 [M+Na]⁺.

### Compound 1-7:

Compound **1-6** (1.60 g, 2.0 mmol) was dissolved in methanol (16 mL), and palladium/carbon (0.20 g) was then added. The mixture was left to react overnight at room temperature in a hydrogen atmosphere. After LCMS analysis showed that the reaction was complete, the reaction mixture was filtered through diatomaceous earth. The filtrate was concentrated to give compound **1-7** (1.0 g, yield: 94%). LCMS:MS (ESI) m/z = 521.9 [M+H]⁺.

### Compound 1-8:

Compound **1-7** (1.20 g, 2.3 mmol) was dissolved in a solution of N,N-dimethylformamide (12 mL), and palmitic acid (1.18 g, 4.6 mmol), O-(benzotriazol-1-yl)-N,N,N',N'-tetramethyluronium tetrafluoroborate (1.55 g, 4.8 mmol), and N,N-diisopropylethylamine (0.89 g, 6.9 mmol) were then added. The mixture was left to react at room temperature for 3 h. After LCMS analysis showed that the reaction was complete, the reaction mixture was poured into water (50 mL) to quench the reaction and extracted with ethyl acetate (50 mL × 2). The organic phases were combined, then washed with saturated brine, dried over anhydrous sodium sulfate, and concentrated, and the resulting crude product was purified and separated by normal-phase silica gel column chromatography to give compound **1-8** (0.70 g, yield: 31%). LCMS:MS (ESI) m/z = 1020.1 [M+Na]⁺.

### Compound A01

Compound **1-8** (0.10 g, 0.1 mmol) was dissolved in anhydrous pyridine (2 mL) and N,N-dimethylformamide (1 mL), and 4-dimethylaminopyridine (0.06 g, 0.5 mmol) and succinic anhydride (0.10 g, 1.0 mmol) were sequentially added. The reaction mixture was stirred at 50 °C for 16 h. After LCMS showed that the reaction was complete, the reaction mixture was poured into water and extracted with ethyl acetate. The extract was washed with saturated brine and dried over anhydrous sodium sulfate to give a crude product of compound **A01** (0.10 g, yield: 95%). LCMS: MS (ESI) m/z = 1096.3 [M-1]⁺.

### Example 2: Synthesis of Compound A02

The synthesis scheme for compound **A02** is as follows:

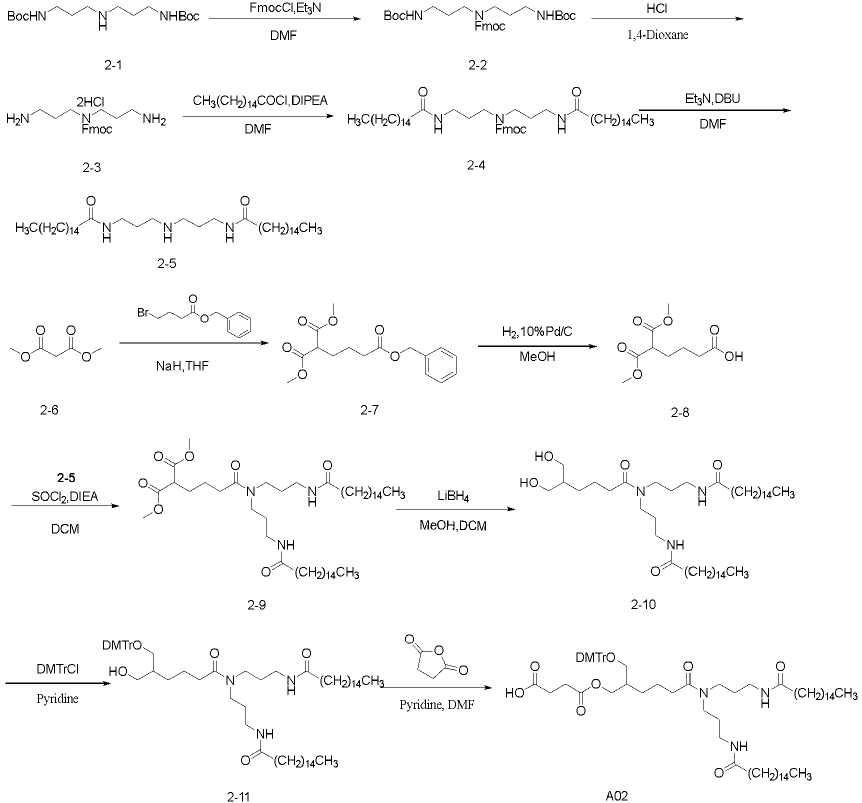

### Compound 2-2:

At 0 °C, 1,9-bis-BOC-1,5,9-triazanonane (10.00 g, 30.0 mmol) was dissolved in a solution of N,N-dimethylformamide (80.0 mL), and triethylamine (9.16 g, 90.0 mmol) and 9-fluorenylmethyl chloroformate (11.70 g, 45.0 mmol) were then added. The mixture was warmed to room temperature and left to react for 18 h. After LC-MS analysis showed that the reaction was complete, the reaction mixture was quenched and diluted with water. The reaction mixture was extracted three times with dichloromethane (80.0 mL). The combined organic phases were washed with saturated brine, dried over anhydrous sodium sulfate, filtered, and concentrated, and the resulting crude product was purified by normal-phase silica gel column chromatography (petroleum ether/ethyl acetate = 10/1) and concentrated under reduced pressure to give compound **2-2** (11.60 g, yield: 69%). LCMS: MS (ESI) m/z = 573.6 [M+Na]⁺.

### Compound 2-3:

Compound **2-2** (11.60 g, 21.0 mmol) was dissolved in dioxane (40.0 mL), and a 4 N solution of hydrochloric acid in dioxane (40.0 mL) was added at room temperature. The mixture was left to react at room temperature for 18 h, and LCMS monitoring showed that the reaction was complete. The reaction mixture was concentrated, and the resulting crude product was triturated (petroleum ether/ethyl acetate = 10/1) to give compound **2-3** (7.41 g, yield: 83%). LCMS: MS (ESI) m/z = 354.5 [M+H]⁺.

### Compound 2-4:

Compound **2-3** (7.20 g, 17.0 mmol) was suspended in N,N-dimethylformamide (150 mL), and diisopropylethylamine (13.00 g, 102.0 mmol) and palmitoyl chloride (14.00 g, 51.0 mmol) were added at room temperature. The mixture was heated to 50 °C and left to react for 18 h, and LCMS monitoring showed that the reaction was complete. Water was added to quench the reaction, and the reaction mixture was extracted three times with dichloromethane (80 mL). The combined organic phases were washed with saturated brine, dried over anhydrous sodium sulfate, and concentrated, and the resulting crude product was triturated (petroleum ether/ethyl acetate = 5/1) to give compound **2-4** (13.60 g, yield: 97%). LCMS: MS (ESI) m/z = 831.4 [M+H]⁺.

### Compound 2-5:

Compound **2-4** (13.60 g, 16.0 mmol) was suspended in N,N-dimethylformamide (130.0 mL), and triethylamine (40.0 mL) was added at room temperature. The mixture was left to react at room temperature for 18 h, and LCMS monitoring showed that about 30% of the starting material remained. Another 10.0 mL of 1,8-diazacyclo[5,4,0]undecene was added, the mixture was heated to 50 °C and left to react for 2 h, and LCMS monitoring showed that the reaction was complete. Water was added to quench the reaction, and the reaction mixture was extracted three times with dichloromethane (80 mL). The combined organic phases were washed with saturated brine, dried over anhydrous sodium sulfate, and concentrated, and the resulting crude product was triturated (pure ethyl acetate) to give compound **2-5** (9.40 g, yield: 94%).

### Compound 2-7:

Dimethyl malonate (8.22 g, 62.2 mmol) was dissolved in anhydrous tetrahydrofuran (80.0 mL), and sodium hydride (2.33 g, 58.3 mmol, 60% in mineral oil) was added at 0 °C. After the mixture was left to react at 0 °C for 1 h, benzyl 4-bromo-n-butyrate (10.0 g, 38.9 mmol) was added dropwise. The reaction mixture was warmed to room temperature and then stirred for 18 h, and LCMS monitoring showed that the reaction was complete. Water was added to quench the reaction, and extraction was performed three times with ethyl acetate (50 mL). The combined organic phases were washed with saturated brine, dried over anhydrous sodium sulfate, and concentrated, and the resulting crude product was purified by normal-phase silica gel column chromatography (petroleum ether/ethyl acetate = 20/1) to give compound **2-7** (6.30 g, yield: 52%). LCMS: MS (ESI) m/z = 309.3 [M+H]⁺.

### Compound 2-8:

Compound **2-7** (8.77 g, 28.4 mmol) was dissolved in methanol (70.0 mL), and 10% palladium on carbon (900 mg) was added at room temperature. The mixture was left to react at room temperature for 18 h in a hydrogen atmosphere, and LCMS monitoring showed that the reaction was complete. The reaction mixture was filtered and concentrated to give compound **2-8** (6.15 g, yield: 99%).

### Compound 2-9:

Compound **2-8** (1.08 g, 4.9 mmol) was dissolved in a solution of thionyl chloride (6.0 mL), and one drop of N,N-dimethylformamide was added at room temperature. After 2 h of refluxing, the reaction mixture was concentrated to give a crude product. The crude product was redissolved in anhydrous dichloromethane (10.0 mL), and the solution was added dropwise to a suspension of compound **2-5** (3.00 g, 4.9 mmol) and N,N-diisopropylethylamine (1.28 g, 9.8 mmol) in anhydrous dichloromethane (50.0 mL). The mixture was left to react at room temperature for 18 h, and LCMS monitoring showed that the reaction was complete. The reaction mixture was quenched with water and extracted three times with dichloromethane (50 mL). The combined organic phases were washed with saturated brine, dried over anhydrous sodium sulfate, and concentrated, and the resulting crude product was purified by normal-phase silica gel column chromatography (dichloromethane/methanol = 20/1) to give compound **2-9** (3.19 g, yield: 80%). LCMS: MS (ESI) m/z = 806.98 [M-H]⁻.

### Compound 2-10:

Compound **2-9** (3.19 g, 3.9 mmol) was dissolved in methanol (15.0 mL) and dichloromethane (15.0 mL), and lithium borohydride (1.74 g, 79.0 mmol) was added at room temperature. The mixture was left to react at room temperature for 18 h, and LCMS monitoring showed that the reaction was complete. The reaction mixture was quenched with water and extracted three times with dichloromethane (50 mL). The combined organic phases were washed with saturated brine, dried over anhydrous sodium sulfate, and concentrated, and the resulting crude product was purified by normal-phase silica gel column chromatography (dichloromethane/methanol = 10/1) to give compound **2-10** (2.22 g, yield: 75%). LCMS: MS (ESI) m/z = 750.96 [M-H]⁻.

### Compound 2-11:

Compound **2-10** (0.21 g, 0.3 mmol) was dissolved in pyridine (5.0 mL), and 4,4'-dimethoxytrityl chloride (0.12 g, 0.3 mmol) was added at room temperature. After the reaction was stirred for 18 h, LCMS monitoring showed that the reaction was complete. The reaction mixture was quenched with water and extracted three times with dichloromethane (50 mL). The combined organic phases were washed with saturated brine, dried over anhydrous sodium sulfate, and concentrated, and the resulting crude product was purified by normal-phase silica gel column chromatography (0.5% triethylamine/dichloromethane/methanol = 10/1) to give compound 2-11 (0.07 g, yield: 24%). LCMS: MS (ESI) m/z = 1053.2 [M-H]⁻. ¹H NMR (400 MHz, CDCl3) δ 7.29 (d, 5H), 7.17 (d, 4H), 6.87-6.79 (m, 4H), 3.88-3.76 (m, 6H), 3.71 (dd, 2H), 3.40 (t, 2H), 3.28 (p, 4H), 3.16 (d, 2H), 2.36 (t, 2H), 2.19 (t, 4H), 2.12-1.96 (m, 3H), 1.82 (p, 2H), 1.76-1.55 (m, 9H), 1.42-1.19 (m, 50H), 0.88 (t, 6H).

### Compound A02:

Compound **2-11** (45 mg, 0.04 mmol) was dissolved in anhydrous pyridine (2 mL) and N,N-dimethylformamide (1 mL), and 4-dimethylaminopyridine (26 mg, 0.21 mmol) and succinic anhydride (43 mg, 0.43 mmol) were sequentially added. The reaction mixture was stirred at 50 °C for 16 h. After LCMS showed that the reaction was complete, the reaction mixture was concentrated, and the resulting crude product was triturated three times with ethyl acetate to give compound **A02** (44 mg, yield: 89%). LCMS: MS (ESI) m/z = 1153.2 [M-H]⁻.

### Example 3: Synthesis of Compound A03

The synthesis scheme for compound A03 is as follows:

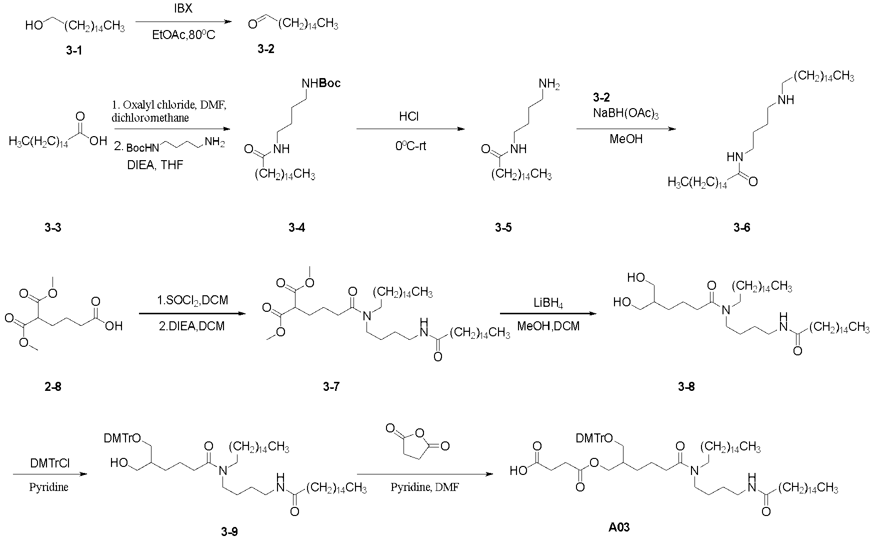

### Compound 3-2:

The compound hexadecanol (5.00 g, 20.6 mmol) was dissolved in ethyl acetate (55 mL), and 2-iodoxybenzoic acid (17.32 g, 61.8 mmol) was added at room temperature. The reaction mixture was heated to 80 °C and stirred for 16 h, and TLC monitoring showed that the reaction was complete. The reaction mixture was cooled to room temperature and filtered, and the filter cake was rinsed with dichloromethane (80 mL). The filtrate was concentrated, and the resulting crude product was purified by normal-phase silica gel column chromatography (petroleum ether/ethyl acetate = 90/10) to give compound **3-2** (4.3 g, yield: 78%). ¹H NMR (400 MHz, CDCl₃) δ 9.77 (t, *J* = 1.6 Hz, 1H), 2.44-2.40 (m, 2H), 1.65-1.61 (m, 2H), 1.26 (s, 24H), 0.88 (t, *J* = 6.8 Hz, 3H).

### Compound 3-4:

At 0 °C, a catalytic amount of N,N-dimethylformamide (one drop) and oxalyl chloride (20.0 mL, 232.8 mmol) were added to a solution of palmitic acid (13.60 g, 53.1 mmol) in dichloromethane (50.0 mL). The reaction was warmed to room temperature and stirred for 2 h. After TLC analysis showed that the reaction was complete, the reaction mixture was concentrated under reduced pressure to give a crude product (14.00 g). At 0 °C, triethylamine (10.75 g, 106.2 mmol) and a solution of palmitoyl chloride (14.00 g) in tetrahydrofuran (20.0 mL) were separately added to a solution of N-tert-butoxycarbonyl-1,4-butanediamine (10.00 g, 53.1 mmol) in tetrahydrofuran (100.0 mL). The reaction mixture was stirred at room temperature for 2 h. LCMS monitoring showed that the reaction was complete. Water was added to quench the reaction, and extraction was performed three times with dichloromethane (80 mL). The combined organic phases were washed with saturated brine, dried over anhydrous sodium sulfate, and concentrated, and the resulting crude product was purified by trituration with ethyl acetate to give compound **3-4** (19.00 g, yield: 75%). LCMS: MS (ESI) m/z = 449.5 [M+Na]⁺.

### Compound 3-5:

At room temperature, a 4 N solution of hydrochloric acid in dioxane (40.0 mL) was added to a solution of compound **3-4** (7.00 g, 16.4 mmol) in dioxane (60.0 mL). After the reaction was stirred for 16 h, LCMS monitoring showed that the reaction was complete. The reaction mixture was concentrated to give compound **3-5** (5.00 g, yield: 75%). LCMS: MS (ESI) m/z = 349.5 [M+Na]⁺.

### Compound 3-6:

At room temperature, compound **3-2** (3.24 g, 13.4 mmol) was added to a suspension of compound **3-5** (4.00 g, 12.2 mmol) in methanol (80 mL), and a few drops of acetic acid and sodium triacetoxyborohydride (5.19 g, 24.5 mmol) were added. The reaction was stirred for 16 h. TLC monitoring showed that the reaction was complete. The reaction mixture was filtered and rinsed with methanol to give compound **3-6** (5.60 g, yield: 78%). ¹H NMR (400 MHz, MeOD) δ 3.20 (t, *J* = 6.8 Hz, 2H), 3.02-2.95 (m, 4H), 2.17 (t, *J* = 7.2 Hz, 2H), 1.70-1.54 (m, 8H), 1.29 (s, 52H), 0.90 (t, *J* = 6.8 Hz, 6H).

### Compound 3-7:

At room temperature, one drop of N,N-dimethylformamide was added to a solution of compound **2-8** (0.74 g, 3.4 mmol) in thionyl chloride (5.0 mL), and the reaction was refluxed for 2 h. TLC monitoring showed that the reaction was complete. The reaction mixture was concentrated, and the resulting crude product was redissolved in anhydrous dichloromethane (10.0 mL). A suspension of compound **3-6** (1.50 g, 2.7 mmol) in anhydrous dichloromethane (50.0 mL) and N,N-diisopropylethylamine (0.88 g, 6.8 mmol) were sequentially added dropwise to this solution. The reaction was stirred at room temperature for 18 h, and LCMS monitoring showed that the reaction was complete. Water was added to quench the reaction, and extraction was performed three times with dichloromethane (80 mL). The combined organic phases were washed with saturated brine, dried over anhydrous sodium sulfate, and concentrated, and the resulting crude product was purified by normal-phase silica gel column chromatography (dichloromethane/methanol = 20/1) to give compound **3-7** (1.53 g, yield: 60%). LCMS: MS (ESI) m/z = 748.0 [M-H]⁻.

### Compound 3-8:

At room temperature, lithium borohydride (0.56 g, 24.4 mmol) was added to a mixed solution of compound **3-7** (1.53 g, 2.0 mmol) in methanol (15.0 mL) and dichloromethane (15.0 mL). The reaction was stirred for 18 h, and LCMS monitoring showed that the reaction was complete. Water was added to quench the reaction, and extraction was performed three times with dichloromethane (80 mL). The combined organic phases were washed with saturated brine, dried over anhydrous sodium sulfate, and concentrated, and the resulting crude product was purified by normal-phase silica gel column chromatography (dichloromethane/methanol = 10/1) to give compound **3-8** (0.91 g, yield: 64%). LCMS: MS (ESI) m/z = 693.9 [M-H]⁻.

### Compound 3-9:

At room temperature, 4,4'-dimethoxytrityl chloride (0.53 g, 1.6 mmol) was added to a solution of compound **3-8** (0.91 g, 1.3 mmol) in pyridine (10.0 mL). The reaction was stirred for 18 h, and LCMS monitoring showed that the reaction was complete. Water was added to quench the reaction, and extraction was performed three times with dichloromethane (50 mL). The combined organic phases were washed with saturated brine, dried over anhydrous sodium sulfate, and concentrated, and the resulting crude product was purified by normal-phase silica gel column chromatography (0.5% triethylamine/dichloromethane/methanol = 10/1) to give compound **3-9** (0.55 g, yield: 42%). LCMS: MS (ESI) m/z = 996.2 [M-H]⁻. 1H NMR (400 MHz, DMSO) δ7.33-7.13 (m, 9H), 6.90-6.83 (m, 4H), 4.35 (td, 1H), 3.72 (d, 6H), 3.42 (tt, 2H), 3.15 (h, 4H), 2.98 (m, 4H), 2.43 (d, 1H), 2.22-2.11 (m, 2H), 2.02 (t, 2H), 1.52-1.10 (m, 62H), 0.89-0.78 (m, 6H).

### Compound A03:

Compound **3-9** (0.15 g, 0.2 mmol) was dissolved in anhydrous pyridine (2 mL) and N,N-dimethylformamide (1 mL), and 4-dimethylaminopyridine (0.19 g, 1.6 mmol) and succinic anhydride (0.08 g, 0.8 mmol) were sequentially added. The reaction mixture was stirred at 50 °C for 16 h. After LCMS showed that the reaction was complete, the reaction mixture was concentrated, and the resulting crude product was triturated three times with ethyl acetate to give compound **A03** (77 mg, yield: 45%). LCMS: MS (ESI) m/z = 1096.2 [M-H]⁻.

### Example 4: Synthesis of Compound A04

The synthesis scheme for compound **A04** is as follows:

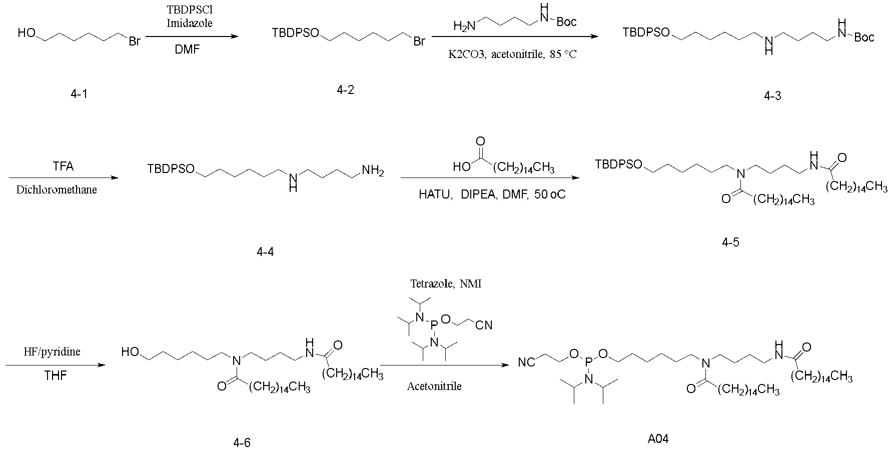

The synthesis and identification of specific intermediates and final products involved in the above scheme are as follows:

### Compound 4-2:

At 0 °C, in a nitrogen atmosphere, tert-butyl(chloro)diphenylsilane (11.38 g, 41.4 mmol) and imidazole (2.82 g, 41.4 mmol) were added to a solution of compound **4-1** (5.00 g, 27.6 mmol) in N,N-dimethylformamide (40 mL). The reaction was warmed to room temperature and stirred for 4 h, and TLC analysis showed that the reaction was complete. The reaction mixture was extracted with petroleum ether (40 mL × 3). The combined organic phases were washed with saturated brine, dried over anhydrous sodium sulfate, and concentrated, and the resulting crude product was purified by normal-phase silica gel column chromatography (pure petroleum ether) to give compound 4-2 (10.0 g, yield: 86%). ¹H NMR (400 MHz, DMSO-d6) δ 7.62 (dd, 4H), 7.49-7.38 (m, 6H), 3.64 (t, 2H), 3.48 (t, 2H), 1.76 (q, 2H), 1.52 (q, 2H), 1.35 (p, 4H), 0.99 (s, 9H).

### Compound 4-3:

N-Boc-butanediamine (13.46 g, 71.52 mmol) and potassium carbonate (9.88 g, 71.5 mmol) were added to a solution of compound 4-2 (10.0 g, 23.8 mmol) in anhydrous acetonitrile (150 mL). The reaction was heated to 85 °C and stirred for 16 h. After LCMS analysis showed that the reaction was complete, the reaction mixture was concentrated, and the resulting crude product was purified by normal-phase silica gel column chromatography (dichloromethane/methanol = 20/1) to give compound **4-3** (5.00 g, yield: 40%). ¹H NMR (400 MHz, DMSO-d6) δ 7.64-7.57 (m, 4H), 7.48-7.38 (m, 6H), 6.84 (t, 1H), 3.63 (t, 2H), 2.90 (q, 2H), 2.51 (p, 1H), 2.43 (q, 4H), 1.52 (m, 2H), 1.37 (s, 19H), 0.99 (s, 9H).

### Compound 4-4:

At 0 °C, a 1 N solution of hydrochloric acid in dioxane (5 mL) was added to a solution of compound **4-3** (5.00 g, 9.5 mmol) in dichloromethane (40 mL). The reaction was warmed to room temperature and stirred for 6 h. After LCMS analysis showed that the reaction was complete, the reaction mixture was concentrated, and the resulting crude product was triturated with petroleum ether to give compound **4-4** (3.20 g, yield: 79%). LCMS: MS (ESI) m/z = 427.5 [M+H]⁺.

### Compound 4-5:

At 0 °C, in a nitrogen atmosphere, O-(7-azabenzotriazol-1-yl)-N,N,N',N'-tetramethyluronium hexafluorophosphate (2.81 g, 7.4 mmol) and N,N-diisopropylethylamine (2.73 g, 21.1 mmol) were added to a solution of palmitic acid (1.89 g, 7.4 mmol) in N,N-dimethylformamide (20 mL). After 0.5 h of stirring at room temperature, compound **4-4** (1.50 g, 3.5 mmol) was added. The reaction was heated to 50 °C and stirred for 16 h. LCMS analysis showed that the reaction was complete. Water was added to quench the reaction, and extraction was performed with dichloromethane (20 mL × 3). The organic phases were combined, then washed with saturated brine, dried over anhydrous sodium sulfate, and concentrated, and the resulting crude product was purified by reversed-phase column chromatography (acetonitrile/water) to give compound **4-5** (1.60 g, yield: 50%). LCMS: MS (ESI) m/z = 903.7 [M+H]⁺

### Compound 4-6:

A solution of pyridine in hydrogen fluoride (3 mL) was added to a solution of compound **4-5** (1.50 g, 1.7 mmol) in tetrahydrofuran (15 mL). The reaction was stirred for 16 h in a nitrogen atmosphere. LCMS analysis showed that the reaction was complete. The reaction mixture was concentrated, and the resulting crude product was triturated with acetonitrile. After filtration, the resulting filter cake was purified by normal-phase silica gel column chromatography (dichloromethane/methanol = 20/1) to give compound **4-6** (0.78 g, yield: 71%). LCMS: MS (ESI) m/z = 663.9 [M-H]⁻.

### Compound A04:

In a nitrogen atmosphere, a 4A molecular sieve was added to a solution of compound **4-6** (0.65 g, 0.6 mmol) in dry dichloromethane (10 mL). After 5 min of stirring at room temperature, a pre-formulated solution of 1H-tetrazole (0.45 M) and N-methylimidazole (0.18 M) in dry acetonitrile (2.3 mL) and bis(diisopropylamino)(2-cyanoethoxy)phosphine (0.70 g, 2.4 mmol) were sequentially added. The mixture was left to react at room temperature for 1 h. After LCMS analysis showed that the reaction was complete, a saturated aqueous sodium bicarbonate solution (10 mL) was added, and extraction was performed with dichloromethane (10 mL × 3). The combined organic phases were washed once with saturated brine (10 mL), dried over anhydrous sodium sulfate, and concentrated under reduced pressure, and the resulting crude product was triturated with acetonitrile. After filtration, the filter cake was washed three times with acetonitrile to give compound **A04** (0.70 g, yield: 82%). LCMS: MS (ESI) m/z = 727.8 [M-137]⁻. ³¹P NMR: (400 MHz, CDCl₃) δ 147.31, 147.16.

### Example 5: Synthesis of Compound A05

The synthesis scheme for compound **A05** is as follows:

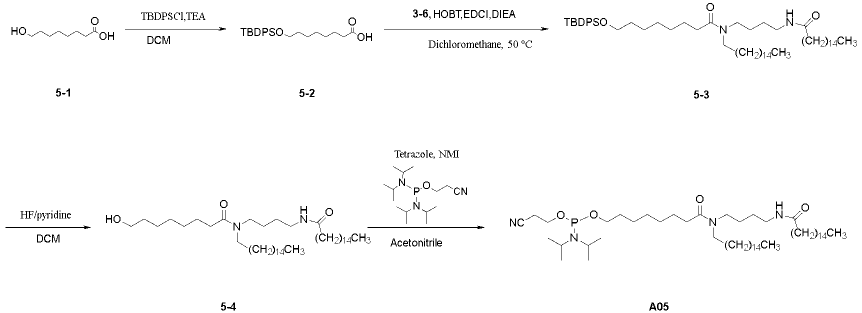

The synthesis and identification of specific intermediates and final products involved in the above scheme are as follows:

### Compound 5-2:

Imidazole (3.19 g, 46.8 mmol) and tert-butyl(chloro)diphenylsilane (9.01 g, 32.8 mmol) were added to a solution of 8-hydroxyoctanoic acid (5.00 g, 31.2 mmol) in dichloromethane (150.0 mL). The reaction was stirred for 2 h, and TLC monitoring showed that the reaction was complete. Water was added to quench the reaction. The separated organic phase was washed with saturated brine, dried over anhydrous sodium sulfate, and concentrated, and the resulting crude product was purified by normal-phase silica gel column chromatography (petroleum ether/ethyl acetate = 6/1) to give compound **5-2** (4.50 g, yield: 36%). ¹H NMR (400 MHz, CDCl₃) δ 7.69-7.65 (m, 4H), 7.46-7.36 (m, 6H), 3.63 (t, 2H), 2.47 (t, 2H), 1.87 (s, 2H), 1.72-1.63 (m, 2H), 1.62-1.52 (m, 2H), 1.39-1.35 (m, 2H), 1.34-1.32 (m, 2H), 1.10 (s, 9H).

### Compound 5-3:

At 0 °C, N,N-diisopropylethylamine (3.0 mL, 18.1 mmol), 1-hydroxybenzotriazole (1.47 g, 10.9 mmol), and 1-(3-dimethylaminopropyl)-3-ethylcarbodiimide hydrochloride (2.09 g, 10.9 mmol) were added to a solution of compound **5-2** (2.89 g, 1.2 mmol) in dichloromethane (60 mL). After the reaction mixture was stirred for 0.5 h, compound **3-6** (2.00 g, 3.6 mmol) was added. Subsequently, the mixture was heated to 50 °C and stirred for 16 h, and LCMS monitoring showed that the reaction was complete. Water was added to quench the reaction. The separated organic phase was washed with saturated brine, dried over anhydrous sodium sulfate, and concentrated, and the resulting crude product was purified by normal-phase silica gel column chromatography (dichloromethane/methanol = 50/1) to give compound 5-3 (1.80 g, yield: 53%). LCMS: MS (ESI) m/z = 932.1 [M+H]⁺.

### Compound 5-4:

At 0 °C, pyridine hydrofluoride (4.00 mL, 44.4 mmol) was added to a solution of compound **5-3** (1.80 g, 1.9 mmol) in dichloromethane (20 mL). The reaction was warmed to room temperature and stirred for 16 h. After LCMS monitoring showed that the reaction was complete, a saturated sodium bicarbonate solution was added to quench the reaction. The separated organic phase was washed with a saturated sodium bicarbonate solution (50 mL × 3), dried over anhydrous sodium sulfate, and concentrated, and the resulting crude product was purified by normal-phase silica gel column chromatography (dichloromethane/methanol = 25/1) to give compound **5-4** (0.89 g, yield: 66%). LCMS: MS (ESI) m/z = 693.9 [M+H]⁺. ¹H NMR (400 MHz, CDCl₃) δ 3.64 (t, 2H), 3.32-3.19 (m, 6H), 2.33 (t, 2H), 2.25-2.17 (m, 2H), 1.98 (s, 4H), 1.67-1.50 (m, 12H), 1.30-1.25 (m, 52H), 0.88 (t, 6H).

### Compound A05:

In a nitrogen atmosphere, a 4A molecular sieve was added to a solution of compound **5-4** (0.59 g, 0.8 mmol) in dry dichloromethane (18 mL). After 5 min of stirring at room temperature, a pre-formulated solution of 1H-tetrazole (0.45 M) and N-methylimidazole (0.18 M) in dry acetonitrile (1.9 mL) and bis(diisopropylamino)(2-cyanoethoxy)phosphine (0.58 g, 1.9 mmol) were sequentially added. The mixture was left to react at room temperature for 1 h. After LCMS analysis showed that the reaction was complete, a saturated aqueous sodium bicarbonate solution (10 mL) was added, and extraction was performed with dichloromethane (10 mL × 3). The combined organic phases were washed once with saturated brine (10 mL), dried over anhydrous sodium sulfate, and concentrated to give compound **A05** (0.60 g, yield: 79%). LCMS: MS (ESI) m/z = 755.9 [M-137]⁻. ³¹P NMR: (400 MHz, CDCl3) δ 147.17, 147.14.

### Example 6: Synthesis of siRNA Conjugates of Present Disclosure

### 6-1. Synthesis of siRNA conjugates containing lipid compound A04'' or A05''

The synthesis of the siRNA conjugates of the present disclosure does not differ from the conventional phosphoramidite solid-phase synthesis method. The synthesis process is briefly described below: Unmodified nucleoside phosphoramidite monomers or nucleoside phosphoramidite monomers having modifications at corresponding positions in the sequence were linked one by one according to a synthesis program on a Dr. Oligo48 synthesizer (Biolytic), with a universal solid-phase support as a start. Nucleoside phosphoramidite monomers, such as 2'-F RNA, 2'-O-methyl RNA, and VPUm monomers, were purchased from Shanghai Hongene or Jiangsu Synthgene Biotechnology Co., Ltd. For siRNA conjugates containing lipid compound A04'' or A05'', the phosphoramidite monomers A04 and A05 prepared in Examples 4 and 5 were used at their corresponding positions, respectively. 5-Ethylthio-1H-tetrazole (ETT) was used as an activator (0.6 M in acetonitrile), a 0.22 M solution of PADS in acetonitrile and 3-methylpyridine (1:1 by volume) (Shanghai Lingjiang) was used as a sulfurizing agent, and an aqueous iodopyridine solution (Shanghai Lingjiang) was used as an oxidant.

After solid-phase synthesis was completed, the oligoribonucleotides were cleaved from the solid support by soaking in a solution of 28% ammonia water and ethanol (3:1) at 50 °C for 16 h. Centrifugation was then performed, and the supernatant was transferred to another centrifuge tube. After the supernatant was concentrated to dryness by evaporation, the residue was purified by C8 reversed-phase chromatography with 0.1 M TEAB and water as the mobile phase. The target oligonucleotides were collected, then lyophilized, identified as the target products by LC-MS, and quantified by UV (260 nm). The single-stranded oligonucleotides obtained were complementarily paired in an equimolar ratio and annealed. The finally obtained double-stranded RNAi agents were dissolved in 1× PBS, and the concentration was adjusted to the concentration required for the experiment so that they were ready for use.

### 6-2. Synthesis of siRNA conjugates containing lipid compound A01", A02", A03", or A06"

The carboxylic acid group-containing compound A01, A02, or A03 (0.1 mmol) was dissolved in anhydrous N,N-dimethylformamide (9.7 mL). After the substrate was almost dissolved, anhydrous acetonitrile (6.5 mL), N,N-diisopropylethylamine (3.0 eq), and N,N,N',N'-tetramethyl-O-(1H-benzotriazol-1-yl)uronium hexafluorophosphate (1.5 eq) were sequentially added. After the reaction mixture was mixed well, macroporous aminomethyl resin (800 mg, target loading: 0.1 mmol/g) was added. The reaction mixture was shaken overnight on a shaker (temperature: 25 °C, rotational speed: 200 rpm). The reaction mixture was filtered, and the filter cake was washed with dichloromethane and then with anhydrous acetonitrile. The solid was collected and dried overnight *in vacuo.* The solid from the previous step was dispersed in anhydrous acetonitrile (5 mL), and a mixed solution of CapB1 (5.0 mL) was added. The reaction mixture was shaken on a shaker (temperature: 25 °C, rotational speed: 200 rpm) for 2 h. The reaction mixture was filtered, and the filter cake was washed with anhydrous acetonitrile. The solid was collected and dried overnight *in vacuo* to give a support-containing resin A01', A02', or A03'. The loading measured 0.1 mmol/g. A support-containing resin A06' can be prepared according to the method disclosed in Patent No. WO2019232255(A1).

siRNA conjugates containing lipid compound A01", A02", A03", or A06" were prepared according to the method described in Example 6-1. The only difference was that the support-containing resin A01', A02', A03', or A06' prepared in the aforementioned step was used instead of the conventional solid-phase support.

**Table 1**

| **Structures of lipid compound monomers or resin-linked lipid compounds** | **Structures of lipid compounds after linking to nucleic acid** |
|---|---|
| **A01'** | **A01"** |
| 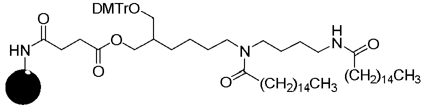 | 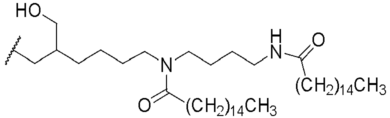 |
| **A02'** | **A02"** |
| 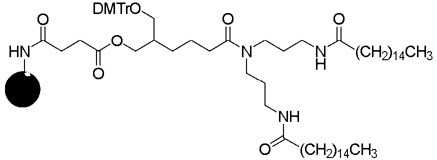 | 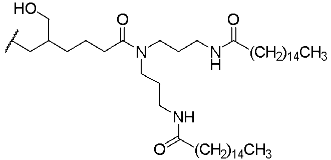 |
| **A03'** | **A03"** |
| 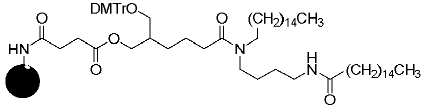 | 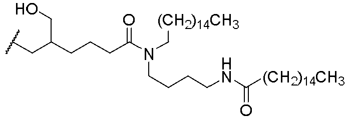 |
| **A04** | **A04"** |
| 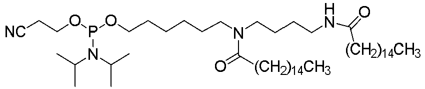 | 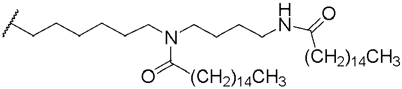 |
| **A05** | **A05"** |
| 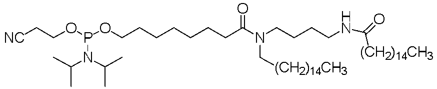 | 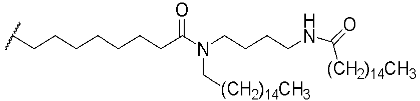 |
| **A06^{'}** | **A06"** |
| 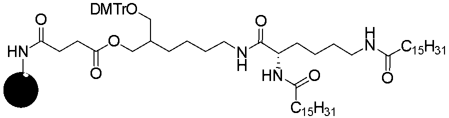 | 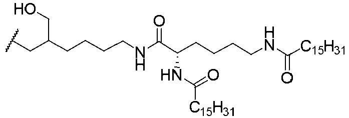 |

In the structures shown in Table 1 above, 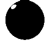 represents macroporous aminomethyl resin.

**Table 2. The sequences of siRNA conjugates targeting the APP gene**

| Double strand No. | SS strand, sequence direction 5'-3' | AS strand, sequence direction 5'-3' |
|---|---|---|
| DS-01 | CmsCmsGmGmUmCmCfCmAfG fGfUmUmAmUmGmAmCmAms CmsAmA01" | VPUmsGfsUmGmUmCfAmUfAfA mCmCmUmGfGmGfAmCmCmG mGmsAmsUm |
| DS-02 | CmsCmsGmGmUmCmCfCmAfG fGfUmUmAmUmGmAmCmAms CmsAmA02” | VPUmsGfsUmGmUmCfAmUfAfA mCmCmUmGfGmGfAmCmCmG mGmsAmsUm |
| DS-03 | CmsCmsGmGmUmCmCfCmAfG fGfUmUmAmUmGmAmCmAms CmsAmA03” | VPUmsGfsUmGmUmCfAmUfAfA mCmCmUmGfGmGfAmCmCmG mGmsAmsUm |
| DS-04 | A04"CmsCmsGmGmUmCmCfC mAfGfGfUmUmAmUmGmAmC mAmsCmsAm | VPUmsGfsUmGmUmCfAmUfAfA mCmCmUmGfGmGfAmCmCmG mGmsAmsUm |
| DS-05 | A05"CmsCmsGmGmUmCmCfC mAfGfGfUmUmAmUmGmAmC mAmsCmsAm | VPUmsGfsUmGmUmCfAmUfAfA mCmCmUmGfGmGfAmCmCmG mGmsAmsUm |
| DS-06 | CmsCmsGmGmUmCmCfCmAfG fGfUmUmAmUmGmAmCmAms CmsAmA06” | VPUmsGfsUmGmUmCfAmUfAfA mCmCmUmGfGmGfAmCmCmG mGmsAmsUm |

**Table 3. The sequences of siRNA conjugates targeting the MAPT gene**

| Double strand No. | SS strand, sequence direction 5'-3' | AS strand, sequence direction 5'-3' |
|---|---|---|
| DS-07 | UmsUmsCmAmCmUmUmUmAf UfCfAmAmUmAmGmUmUmC msCmsAmA01” | VPUmsGfsGmAmAmCfUmAmU mUmGmAfUmAfAmAfGmUmG mAmAmsUmsUm |
| DS-08 | UmsUmsCmAmCmUmUmUmAf UfCfAmAmUmAmGmUmUmC msCmsAmA02” | VPUmsGfsGmAmAmCfUmAmU mUmGmAfUmAfAmAfGmUmG mAmAmsUmsUm |
| DS-09 | UmsUmsCmAmCmUmUmUmAf UfCfAmAmUmAmGmUmUmC msCmsAmA03” | VPUmsGfsGmAmAmCfUmAmU mUmGmAfUmAfAmAfGmUmG mAmAmsUmsUm |
| DS-10 | A04"UmsUmsCmAmCmUmUm UmAfUfCfAmAmUmAmGmUm UmCmsCmsAm | VPUmsGfsGmAmAmCfUmAmU mUmGmAfUmAfAmAfGmUmG mAmAmsUmsUm |
| DS-11 | A05"UmsUmsCmAmCmUmUm UmAfUfCfAmAmUmAmGmUm UmCmsCmsAm | VPUmsGfsGmAmAmCfUmAmU mUmGmAfUmAfAmAfGmUmG mAmAmsUmsUm |
| DS-12 | UmsUmsCmAmCmUmUmUmAf UfCfAmAmUmAmGmUmUmC msCmsAmA06" | VPUmsGfsGmAmAmCfUmAmU mUmGmAfUmAfAmAfGmUmG mAmAmsUmsUm |

**Table 4. The sequences of siRNA conjugates targeting the SARM1 gene**

| Double strand No. | SS strand, sequence direction 5'-3' | AS strand, sequence direction 5'-3' |
|---|---|---|
| DS-13 | UmsUmsCmGmCmCmAmAmCf UfAfUmUmCmUmAmCmGmU msGmsAmA01" | VPUmsCfsAmCfGmUfAmGmA mAfUmAfGmUfUmGfCmCfGm AmAmsGmsGm |
| DS-14 | UmsUmsCmGmCmCmAmAmCf UfAfUmUmCmUmAmCmGmU msGmsAmA02” | VPUmsCfsAmCfGmUfAmGmA mAfUmAfGmUfUmGfCmCfGm AmAmsGmsGm |
| DS-15 | UmsUmsCmGmCmCmAmAmCf UfAfUmUmCmUmAmCmGmU msGmsAmA03” | VPUmsCfsAmCfGmUfAmGmA mAfUmAfGmUfUmGfCmCfGm AmAmsGmsGm |
| DS-16 | A04"UmsUmsCmGmCmCmAm AmCfUfAfUmUmCmUmAmCm GmUmsGmsAm | VPUmsCfsAmCfGmUfAmGmA mAfUmAfGmUfUmGfCmCfGm AmAmsGmsGm |
| DS-17 | A05"UmsUmsCmGmCmCmAm AmCfUfAfUmUmCmUmAmCm GmUmsGmsAm | VPUmsCfsAmCfGmUfAmGmA mAfUmAfGmUfUmGfCmCfGm AmAmsGmsGm |
| DS-18 | UmsUmsCmGmCmCmAmAmCf UfAfUmUmCmUmAmCmGmU msGmsAmA06” | VPUmsCfsAmCfGmUfAmGmA mAfUmAfGmUfUmGfCmCfGm AmAmsGmsGm |

**Table 5. The sequences of siRNA conjugates targeting the PMP22 gene**

| Double strand No. | SS strand, sequence direction 5'-3' | AS strand, sequence direction 5'-3' |
|---|---|---|
| DS-19 | CmsCmsUmCmCmUmGmUmUf GfCfUmGmAmGmUmAmUmC msAmsAmA01” | VPUmsUfsGmAmUmAfCmUmC mAmGmCmAmAfCmAfGmGmA mGmGmsAmsGm |
| DS-20 | CmsCmsUmCmCmUmGmUmUf GfCfUmGmAmGmUmAmUmC msAmsAmA02” | VPUmsUfsGmAmUmAfCmUmC mAmGmCmAmAfCmAfGmGmA mGmGmsAmsGm |
| DS-21 | CmsCmsUmCmCmUmGmUmUf GfCfUmGmAmGmUmAmUmC msAmsAmA03" | VPUmsUfsGmAmUmAfCmUmC mAmGmCmAmAfCmAfGmGmA mGmGmsAmsGm |
| DS-22 | A04"CmsCmsUmCmCmUmGm UmUfGfCfUmGmAmGmUmAm UmCmsAmsAm | VPUmsUfsGmAmUmAfCmUmC mAmGmCmAmAfCmAfGmGmA mGmGmsAmsGm |
| DS-23 | A05"CmsCmsUmCmCmUmGm UmUfGfCfUmGmAmGmUmAm UmCmsAmsAm | VPUmsUfsGmAmUmAfCmUmC mAmGmCmAmAfCmAfGmGmA mGmGmsAmsGm |

In Tables 2-5 above, the sequences are shown in the direction from the 5' end to the 3' end (from left to right); "G", "C", "A", "T", and "U" represent nucleosides containing the bases of guanine, cytosine, adenine, thymine, and uracil, respectively. The lowercase letter m means that the nucleoside adjacent to the letter m on the left side is a 2'-methoxy-modified nucleoside; the lowercase letter f means that the nucleoside adjacent to the letter f on the left side is a 2'-fluoro-modified nucleoside; VP means that the 5' end of the nucleoside adjacent to the letters on the left/right side is a trans-vinylphosphodiester group (5'-E-VP); A01", A02", A03", A04", A05", and A06" are lipid compounds having the corresponding structures shown in Table 1, respectively; the lowercase letter s means that the two nucleosides adjacent to the letter s, or a nucleoside and a lipid compound, are linked by a phosphorothioate diester group. Unless otherwise specified, two adjacent nucleosides, or a nucleoside or a nucleoside and a lipid compound, are linked by a phosphodiester group. Unless otherwise specified, the 3' position of the first nucleotide at the 3' end of each strand is hydroxy; the 5' position of the first nucleotide at the 5' end of each strand is hydroxy.

The structures of the 2'-methoxy-modified nucleoside, the 2'-fluoro-modified nucleoside, the trans-vinylphosphodiester group (5'-E-VP), the phosphorothioate diester group, and the phosphodiester group are shown in the table below. When an siRNA of the present disclosure is present in the form of a salt, for example, in the form of a sodium salt, the structures of salt forms corresponding to the structures in Table 6 below also fall within the protection scope of the present disclosure. In Table 6, Base represents a base at the corresponding position:

**Table 6**

| Structure | Name |
|---|---|
| 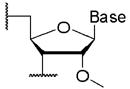 | 2'-Methoxy-modified nucleoside |
| 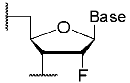 | 2'-Fluoro-modified nucleoside |
| 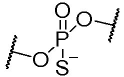 | Anion form of phosphorothioate diester group |
| 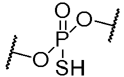 | Phosphorothioate diester group |
| 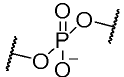 | Anion form of phosphodiester group |
| 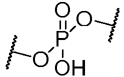 | Phosphodiester group |
| 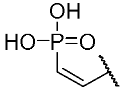 | Z-Vinylphosphoric acid group (Z-VP) |
| 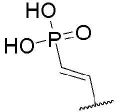 | E-Vinylphosphoric acid group (E-VP) |

### Example 7. In Vitro Knockout Efficiency Evaluation of Conjugates of Present Disclosure in Human A549 and Hek-293A Cells

The *in vitro* APP knockout efficiency of the siRNA conjugates shown in Table 2 was evaluated using 5 concentration gradients in A549 cells or Hek-293A cells. The specific procedure was as follows:
A549 cells (purchased from Cobioer Biosciences, Cat. No. CRM-CCL-185) and Hek-293A cells (purchased from Cobioer Biosciences, Cat. No. CBP60436) were cultured at 37 °C with 5% CO₂ in F-12K and DMEM culture media containing 10% fetal bovine serum, respectively. Twenty-four hours before the free uptake experiment, A549 cells and Hek-293A cells were seeded into 96-well plates at a density of 2 × 10⁴ cells/well and 1 × 10⁴ cells/well, respectively, with each well containing 100 µL of the culture medium. After 48 h of treatment, total cell RNA extraction was performed using an FG0417-L/FG0418-XL high-throughput cell RNA extraction kit (FireGen, magnetic bead method), and RNA reverse transcription (Takara, RR037A) and real-time quantitative PCR (Thermo, 4444557) detection were then performed to determine the mRNA level of human APP. The mRNA level of human APP was corrected based on the GAPDH internal reference gene level.

The instruments involved in the experiment are shown in Table 7.

**Table 7. Experimental instruments**

| Name | Company | Cat. No./model |
|---|---|---|
| Nanodrop | Thermo | Nanodrop One |
| qPCR system | ABI | 7500 Fast |

For the real-time quantitative PCR detection, a probe Q-PCR detection experiment was used, and its primer information is shown in Table 8.

**Table 8. Taqman primers**

| Primer name | Primer sequence (5'-3') |
|---|---|
| hAPP-Forward | GACAGACAGCACACCCTAAA |
| hAPP-Reverse | CACACGGAGGTGTGTCATAA |
| hAPP-Probe | 5`6-FAM-ATCCCAAGAAAGCCGCTCAGATCC-3`BHQ1 |
| hGAPDH-Forward | CCAGGTGGTCTCCTCTGACTTC |
| hGAPDH-Reverse | GTGGTCGTTGAGGGCAATG |
| hGAPDH-Probe | 5'VIC-ACAGCGACACCCACTCCTCCACCTT-3'BHQ1 |

### Result analysis method

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2-△ △Ct, where △△Ct = [(Ct experimental group target gene - Ct experimental group internal reference) - (Ct control group target gene - Ct control group internal reference)]. Inhibition rate (%) = (1 - remaining level of target gene expression) × 100%.

The results are expressed relative to the remaining percentage of human APP mRNA expression in cells treated with the siRNA conjugates.

The data results in Table 9 show that the siRNA conjugates of the present disclosure all exhibited good inhibitory activity against the APP gene in A549 cells and Hek-293A cells.

**Table 9. The APP knockout activity of siRNA conjugates in A549 cells and Hek-293A cells**

| A549 cells | | | | | | |
|---|---|---|---|---|---|---|
| Sequence No. | Remaining percentage of APP mRNA expression (%) | | | | | |
| | 3000 nM | 1000 nM | 333.3 nM | 111.1 nM | 37 nM | 0 nM |
| DS-01 | 7.79 | 29.61 | 52.41 | 70.87 | 90.11 | 96.81 |
| DS-02 | 66.57 | 73.17 | 85.15 | 88.65 | 89.75 | 99.17 |

| Hek-293A cells | | | | | | |
|---|---|---|---|---|---|---|
| Sequence No. | Remaining percentage of APP mRNA expression (%) | | | | | |
| | 3000 nM | 1000 nM | 333.3 nM | 111.1 nM | 37 nM | 0 nM |
| DS-01 | 9.95 | 48.53 | 87.55 | 91.52 | 93.44 | 98.50 |
| DS-02 | 28.46 | 70.57 | 83.65 | 91.05 | 98.15 | 96.41 |

### Example 8: Evaluation of In Vitro Inhibitory Activity of siRNA Conjugates in Mouse Neuro2α Cells

The *in vitro* APP knockout efficiency of the siRNA conjugates shown in Table 2 was evaluated using 2 concentration gradients in Neuro2α cells. The specific procedure was as follows:
Neuro2α cells (purchased from the ATCC cell bank, Cat. No. CCL-131) were cultured at 37 °C with 5% CO₂ in an MEM culture medium containing 10% fetal bovine serum. Twenty-four hours before the free uptake experiment, the Neuro2α cells were seeded into a 96-well plate at a density of 1.5 × 10⁴ cells/well, with each well containing 100 µL of the culture medium. After 72 h of treatment, total cell RNA extraction was performed using an MNTR/FX96 (Lr) high-throughput cell RNA extraction kit (GeneOn BioTech, magnetic bead method), and RNA reverse transcription (Takara, RR037A) and real-time quantitative PCR (Thermo, 4444557) detection were then performed to determine the mRNA level of mouse APP. The mRNA level of mouse APP was corrected based on the GAPDH internal reference gene level.

The instruments involved in the experiment are shown in Table 7.

For the real-time quantitative PCR detection, a probe Q-PCR detection experiment was used, and its primer information is shown in Table 10.

**Table 10. Taqman primers**

| Primer name | Primer sequence (5'-3') |
|---|---|
| Mice APP-Forward | TGACAAGAAGGCCGTTATCC |
| Mice APP-Reverse | GGCCATGTGTGTCTCTACAA |
| Mice APP-Probe | 5`6-FAM-CTGTCTCTCATTGGCTGCTTCCTGTTC-3`BHQ1 |
| Mice GAPDH-Forward | AACAGCAACTCCCACTCTTC |
| Mice-GAPDH-Reverse | CCTGTTGCTGTAGCCGTATT |
| Mice-GAPDH-Probe | 5'VIC- AAAGTTGTCATTGAGAGCAATGCCAGC-3'BHQ1 |

### Result analysis method

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-△△Ct}, where △△Ct = [(Ct experimental group target gene - Ct experimental group internal reference) - (Ct control group target gene - Ct control group internal reference)]. Inhibition rate (%) = (1 - remaining level of target gene expression) × 100%.

The results are expressed relative to the remaining percentage of mouse APP mRNA expression in cells treated with the siRNA conjugates, and "+" means that the remaining percentage of expression is less than or equal to 50%; that is, there is a high level of knockout activity. The data results in Table 11 show that the siRNA conjugates of the present disclosure exhibited high levels of knockout activity against the APP gene in Neuro2α cells.

**Table 11. Inhibitory activity against APP mRNA in Neuro2α cells**

| No. | Remaining percentage of APP mRNA expression (%) | |
|---|---|---|
| | 3000 nM | 1000 nM |
| DS-01 | + | + |

### Example 9. Inhibitory Activity of Conjugates of Present Disclosure Against Mouse APP Gene

Twenty-four C57BL/6J male mice aged 8 weeks (purchased from Zhejiang Vital River Laboratory Animal Technology Co., Ltd.) were acclimatized for one week before the experiment. The compounds were dissolved in normal saline before administration on day 0, and each mouse was dosed at 5 mg/kg. The mice were weighed and divided into 4 groups (6 mice/group), and the 4 groups were given normal saline (blank vehicle group), siRNA conjugate DS-01, siRNA conjugate DS-04, and siRNA conjugate DS-05, respectively. After anesthesia with Zoletil, lateral intracerebroventricular (ICV) administration was performed using a stereotaxic apparatus (RWD). A standard sterile surgical procedure was used to perform a single injection of 2 µL into the right lateral ventricle at the following position: AP = -0.2 mm (posterior to bregma), ML = -1.0 mm, and DV = +2.4 mm. After a midline incision was made in the scalp, a burr hole was created at the selected coordinates using the stereotaxic coordinates and a cranial drill. Subsequently, the dura mater was punctured at the center of the burr hole, and the needle was lowered to the specified depth to reach the lateral ventricle. The glass electrode filling volume was slightly greater than 2 µL. The administration volume was 2 µL, and the injection rate was 1 µL/min. After infusion, the needle was left in place and stabilized for 2 min, and the needle was slowly withdrawn. When the needle tip was within the cortical region, the withdrawal was paused for 30 s. On day 28, the mice were euthanized, and hippocampus, striatum, and spinal cord tissues were collected from the mice, snap-frozen in liquid nitrogen, and then stored at -80 °C. The APP mRNA expression levels in the cerebral cortexes of the mice were determined by RT-PCR.

The experimental results are shown in Table 12 and FIG. 1. Table 12 shows day-28 APP mRNA inhibition rates in mice of the groups given the siRNA conjugates of the present disclosure relative to the blank vehicle group. It can be seen from the results in Table 12 and FIG. 1 that the siRNA conjugates DS-01, DS-04, and DS-05 of the present disclosure all exhibited good inhibitory activity against APP mRNA in different brain regions of mice.

**Table 12. The inhibitory activity of siRNA conjugates of the present disclosure against APP mRNA in mice**

| Group | Hippocampus Inhibition rate (%) | Striatum Inhibition rate (%) | Spinal cord Inhibition rate (%) |
|---|---|---|---|
| DS-01 | 58.9 | 44.9 | 52.3 |
| DS-04 | 58.9 | 62.1 | 61.0 |
| DS-05 | 60.6 | 61.1 | 52.5 |

### Example 10. Inhibitory Activity of Conjugates of Present Disclosure Against Mouse MAPT Gene

Thirty C57BL/6J male mice aged 8 weeks (purchased from Zhejiang Vital River Laboratory Animal Technology Co., Ltd.) were acclimatized for one week before the experiment. The compounds were dissolved in normal saline before administration on day 0, and each mouse was dosed at 5 mg/kg. The mice were weighed and divided into 5 groups (6 mice/group), and the 5 groups were given normal saline (blank vehicle group), siRNA conjugate DS-07, siRNA conjugate DS-10, siRNA conjugate DS-11, and siRNA conjugate DS-12, respectively. After anesthesia with Zoletil, lateral intracerebroventricular (ICV) administration was performed using a stereotaxic apparatus (RWD). A standard sterile surgical procedure was used to perform a single injection of 2 µL into the right lateral ventricle at the following position: AP = -0.2 mm (posterior to bregma), ML = -1.0 mm, and DV = +2.4 mm. After a midline incision was made in the scalp, a burr hole was created at the selected coordinates using the stereotaxic coordinates and a cranial drill. Subsequently, the dura mater was punctured at the center of the burr hole, and the needle was lowered to the specified depth to reach the lateral ventricle. The glass electrode filling volume was slightly greater than 2 µL. The administration volume was 2 µL, and the injection rate was 1 µL/min. After infusion, the needle was left in place and stabilized for 2 min, and the needle was slowly withdrawn. When the needle tip was within the cortical region, the withdrawal was paused for 30 s. On day 28, the mice were euthanized, and hippocampus, striatum, and spinal cord tissues were collected from the mice, snap-frozen in liquid nitrogen, and then stored at -80 °C. The MAPT mRNA levels in the cerebral cortexes of the mice were determined by RT-PCR. The experimental results are shown in Table 13 and FIG. 2.

Table 13 shows day-28 MAPT mRNA inhibition rates in mice of the groups given the siRNA conjugates of the present disclosure and the reference siRNA conjugate relative to the blank vehicle group. The results in Table 13 and FIG. 2 show that the siRNA conjugates DS-07, DS-10, and DS-11 of the present disclosure all exhibited significantly better inhibitory activity against MAPT mRNA than the comparison siRNA conjugate DS-12 in mice.

**Table 13. The inhibitory activity of siRNA conjugates against MAPT mRNA in mice**

| Group | Hippocampus Inhibition rate (%) | Striatum Inhibition rate (%) | Spinal cord Inhibition rate (%) |
|---|---|---|---|
| DS-07 | 41.9 | 23.1 | 26.2 |
| DS-10 | 59.6 | 48.3 | 50.6 |
| DS-11 | 51.4 | 38.9 | 48.3 |
| DS-12 | 21.0 | None | None |

### Example 11: In Vitro Inhibitory Activity of siRNA Conjugates Against Target mRNA in Human A172 Cells

The *in vitro* inhibitory activity of siRNAs of the present disclosure against SARM1 was evaluated using 2 concentration gradients in A172 cells. The specific procedure was as follows:
A172 cells (purchased from Cobioer Biosciences, Cat. No. CBP60575) were cultured at 37 °C with 5% CO₂ in a DMEM culture medium containing 10% fetal bovine serum. Twenty-four hours before the free uptake experiment, the A172 cells were seeded into a 96-well plate at a density of 0.7 × 10⁴ cells/well, with each well containing 100 µL of the culture medium. After 72 h of treatment, total cell RNA extraction was performed using an MNTR/FX96 (Lr) high-throughput cell RNA extraction kit (GeneOn BioTech, magnetic bead method), and RNA reverse transcription (Takara, RR037A) and real-time quantitative PCR (Thermo, 4444557) detection were then performed to determine the mRNA level of human SARM1. The mRNA level of human SARM1 was corrected based on the GAPDH internal reference gene level.

The instruments involved in the experiment are shown in Table 7. For the real-time quantitative PCR detection, a probe Q-PCR detection experiment was used, and its primer information is shown in Table 14.

**Table 14. Taqman primers**

| Primer name | Primer sequence (5'-3') |
|---|---|
| hGAPDH-Forward | CCAGGTGGTCTCCTCTGACTTC |
| hGAPDH-Reverse | GTGGTCGTTGAGGGCAATG |
| hGAPDH-Probe | 5'VIC-ACAGCGACACCCACTCCTCCACCTT-3'BHQ1 |
| hPMP22 -Forward | GTGGCATCTCAACTCGGATTAC |
| hPMP22-Reverse | CGTTTCCGCAAGATCACATAGA |
| hPMP22-Probe | 5`6-FAM-CTACGGTTTCGCCTACATCCTGGC-3`BHQ1 |
| hSARM1-Forward | CTTTAAGCTGCGGCAAGAAC |
| hSARM1-GAPDH-Reverse | ATTCGTGGGACCACTTGATAC |
| hSARM1-GAPDH-Probe | 5`6-FAMCATGCAGGCTGTGCTTACTTTCAACG- 3`BHQ1 |

### Result analysis method

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-△△Ct}, where △△Ct = [(Ct experimental group target gene - Ct experimental group internal reference) - (Ct control group target gene - Ct control group internal reference)]. Inhibition rate (%) = (1 - remaining level of target gene expression) × 100%.

The results are expressed relative to the remaining percentage of human SARM1 mRNA expression in cells treated with the siRNA conjugates. In the results, "+" means that the remaining percentage of expression is less than or equal to 50%; that is, there is a high level of knockout activity.

The data results in Table 15 show that the siRNA conjugates of the present disclosure all exhibited good inhibitory activity against the SARM1 gene in A172 cells.

**Table 15. The inhibitory activity of siRNA conjugates in A172 cells**

| No. | Remaining percentage of SARM1 mRNA expression (%) | |
|---|---|---|
| | 3000 nM | 1000 nM |
| DS-13 | + | + |
| DS-14 | + | + |
| DS-15 | + | + |
| DS-16 | + | + |
| DS-17 | + | + |

### Example 12: In Vitro Inhibitory Activity of siRNA Conjugates in Human HEK293A Cells

The *in vitro* inhibitory activity of siRNA conjugates of the present disclosure against PMP22 mRNA was evaluated using 2 concentration gradients in HEK293A cells. The specific procedure was as follows:
HEK293A cells (purchased from Cobioer Biosciences, Cat. No. CBP60436) were cultured at 37 °C with 5% CO₂ in a DMEM culture medium containing 10% fetal bovine serum. Twenty-four hours before the free uptake experiment, the HEK293A cells were seeded into a 96-well plate at a density of 0.7 × 10⁴ cells/well, with each well containing 100 µL of the culture medium. After 72 h of treatment, total cell RNA extraction was performed using an MNTR/FX96 (Lr) high-throughput cell RNA extraction kit (GeneOn BioTech, magnetic bead method), and RNA reverse transcription (Takara, RR037A) and real-time quantitative PCR (Thermo, 4444557) detection were then performed to determine the mRNA level of human PMP22. The mRNA level of human PMP22 was corrected based on the GAPDH internal reference gene level.

The instruments, primer information, and result analysis method involved in this experiment were the same as those in Example 11. The results are shown in Table 16. In the results, "+" means that the remaining percentage of expression is less than or equal to 50%; that is, there is a high level of knockout activity.

**Table 16. The inhibitory activity of siRNA conjugates in HEK293A cells**

| No. | Remaining percentage of PMP22 mRNA expression (%) | |
|---|---|---|
| | 3000 nM | 1000 nM |
| DS-19 | + | + |
| DS-20 | + | + |
| DS-21 | + | + |

### Example 13: Evaluation of Metabolic Stability of siRNA Conjugates in Various Parts of Mice

Fresh liver tissue samples were collected from C57 male mice (Zhejiang Vital River Laboratory Animal Technology Co., Ltd.), and 1000 µL of a homogenization buffer (100 mM Tris, 1.5 mM MgCl₂, pH 5.9) was added per 200 mg. The samples were left to stand in a freezer at -40 °C for 10 min and then ground in a cell tissue grinder at 4 °C for 3 min (3 cycles) to prepare liver homogenates.

The siRNA conjugates DS-01, DS-07, and DS-13 of the present disclosure and the comparison conjugates DS-06, DS-12, and DS-18 were each dissolved in 30% methanol/water (containing 0.2% diisopropylethylamine) to prepare solutions with a concentration of 50 µM. 150 µL of a liver homogenate was taken, and 4 µL of an siRNA solution described above was added. After thorough mixing, the mixture was incubated at 37 °C for 48 h at a rotational speed of 500 rpm. After 48 h, 150 µL of a liver homogenate was taken, and 4 µL of an siRNA conjugate solution described above was added to obtain a 48-hour incubation sample. 20 µL of an internal standard (siRNA analog) was added to the 0-hour incubation sample or the 48-hour incubation sample. After undergoing solid-phase extraction, the sample was analyzed by LC-MS. The stability of the siRNA conjugates in mouse liver homogenates was expressed in terms of the remaining percentage of the AS strand (% remaining), and was calculated through the ratio of the peak area of the test substance to the peak area of the internal standard after 48 h of incubation/the ratio of the peak area of the test substance to the peak area of the internal standard after 0 h of incubation. DS-01 and DS-06 were both APP-targeting siRNA conjugates and differed only in their structures, DS-07 and DS-12 were both MAPT-targeting siRNA conjugates and differed only in their structures, and DS-13 and DS-18 were both SARM1-targeting conjugates and differed only in their structures.

The results show that the remaining percentage of the APP-targeting siRNA conjugate DS-01 of the present disclosure was 1.28 times that of the comparison conjugate DS-06, the remaining percentage of the MAPT-targeting siRNA conjugate DS-07 of the present disclosure was 1.30 times that of the comparison conjugate DS-12, and the remaining percentage of the SARM1-targeting siRNA conjugate DS-13 of the present disclosure was 1.24 times that of the comparison conjugate DS-18.

The above results indicate that the siRNA conjugates of the present disclosure targeting different targets all exhibited significantly better stability in liver homogenates than the comparison siRNA conjugates. In addition, the stability of the siRNA conjugates of the present disclosure in mouse brain homogenates and mouse serum was also assessed, and they all exhibited excellent stability.

## Claims

1. A conjugate represented by formula I or a pharmaceutically acceptable salt thereof, 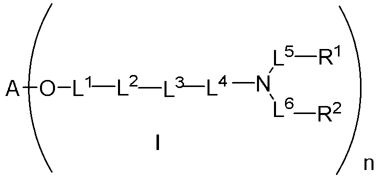
wherein A is a nucleic acid;
-L¹-L²-L³-L⁴- is 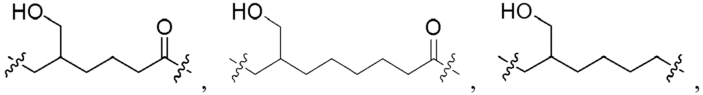 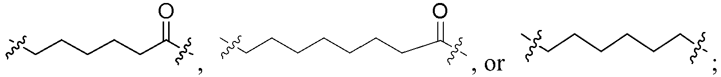
L⁵ and L⁶ are identical or different and are each independently a bond, 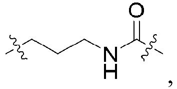 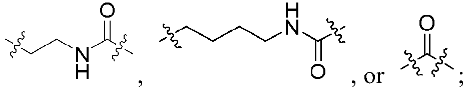
R¹ and R² are each independently -(CH₂)₇CH₃, -(CH₂)₁₂CH₃, -(CH₂)₁₃CH₃, -(CH₂)₁₄CH₃, -(CH₂)₁₅CH₃, -(CH₂)₁₆CH₃, -(CH₂)₁₈CH₃, or -(CH₂)₂₀CH₃;
n is 1 or 2;
preferably, n is 1.

2. The conjugate represented by formula I or the pharmaceutically acceptable salt thereof according to claim 1, wherein the conjugate represented by formula I is a conjugate represented by formula I-1 or I-2: 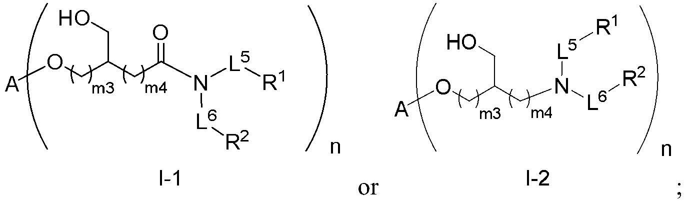
in formula I-1 and formula I-2, m3 is 1, and m4 is 3 or 4;
or the conjugate represented by formula I is a conjugate represented by formula I-3 or I-4: 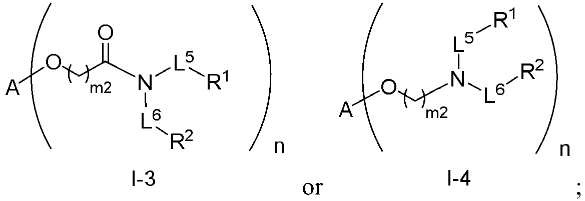
in formula I-3 and formula I-4, m2 is 6 or 7;
R¹, R², A, L⁵, L⁶, and n are as defined in claim 1.

3. The conjugate represented by formula I or the pharmaceutically acceptable salt thereof according to claim 1 or 2, wherein the nucleic acid is an siRNA; in the siRNA, the 3' end of the SS strand or the AS strand is linked to -O-L¹-, or the 5' end of the SS strand or the AS strand is linked to -O-L¹-, or both the 3' end and the 5' end of the SS strand or the AS strand are linked to -O-L¹-.

4. The conjugate represented by formula I or the pharmaceutically acceptable salt thereof according to any one of claims 1-3, wherein the nucleic acid is linked to L¹ by a phosphodiester group, wherein the O atom in -O-L¹- is attributed to the phosphodiester group;
or the nucleic acid is linked to L¹ by a phosphorothioate diester group, wherein the O atom in -O-L¹- is attributed to the phosphorothioate diester group.

5. The conjugate represented by formula I or the pharmaceutically acceptable salt thereof according to any one of claims 1-4, having a structure represented by any one of the following: 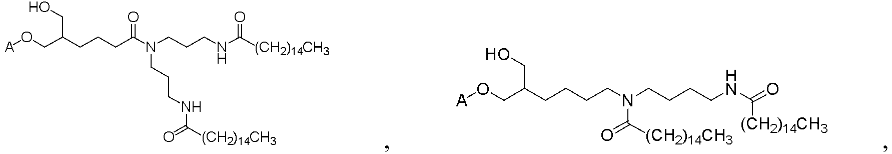 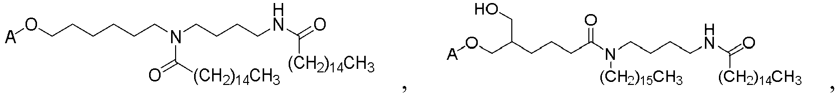 or 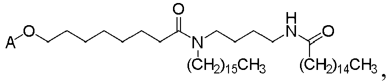 wherein A is as defined in claim 1.

6. A compound represented by formula II or a pharmaceutically acceptable salt thereof, 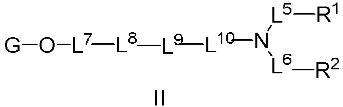
wherein L⁵, L⁶, R¹, and R² are as defined in any one of claims 1-5;
G is hydrogen, 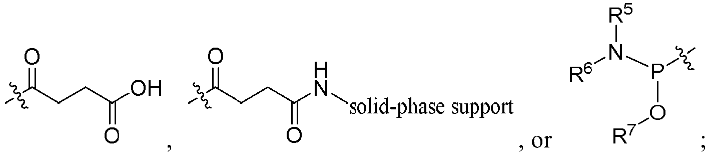
R⁵ and R⁶ are isopropyl, and R⁷ is 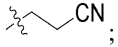
each -L⁷-L⁸-L⁹-L¹⁰- is independently

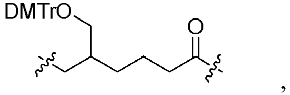

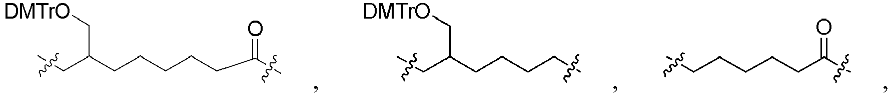

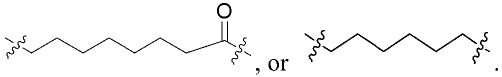

7. The compound represented by formula II or the pharmaceutically acceptable salt thereof according to claim 6, wherein the compound represented by formula II is a compound represented by formula II-1 or II-2: 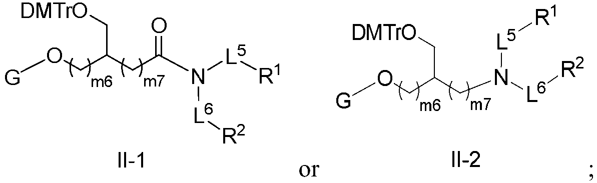
in formulas II-1 and II-2, m6 is 1, and m7 is 3 or 4;
or the compound represented by formula II is a compound represented by formula II-3 or II-4: 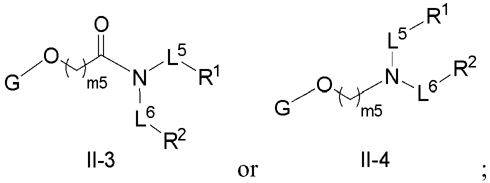
in formulas II-3 and II-4, m5 is 6 or 7;
R¹, R², G, L⁵, and L⁶ are as defined in claim 6.

8. The compound represented by formula II or the pharmaceutically acceptable salt thereof according to claim 7, wherein the compound represented by formula II is a compound represented by formula II-1A or II-2A: 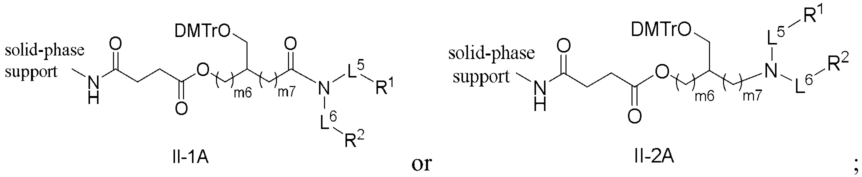
in formulas II-1A and II-2A, m6 is 1, and m7 is 3 or 4;
or the compound represented by formula II is a compound represented by formula II-3A or II-4A: 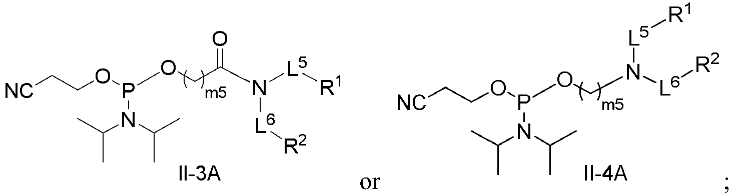
in formulas II-3A and II-4A, m5 is 6 or 7;
R¹, R², L⁵, and L⁶ are as defined in claim 6.

9. A compound of the following structure or a pharmaceutically acceptable salt thereof, wherein 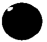 is a solid-phase support: 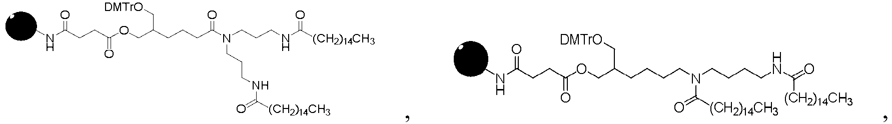 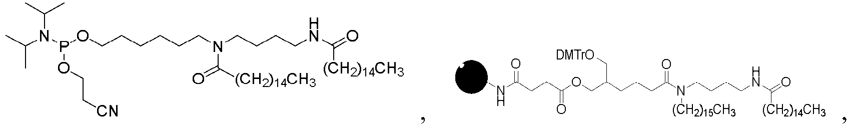 or

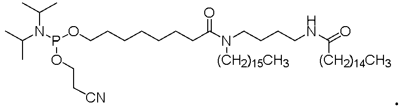

10. An RNAi agent, comprising the conjugate represented by formula I or the pharmaceutically acceptable salt thereof according to any one of claims 1-5.

11. A pharmaceutical composition, comprising the conjugate represented by formula I or the pharmaceutically acceptable salt thereof according to any one of claims 1-5 or the RNAi agent according to claim 10, and one or more pharmaceutically acceptable excipients.

12. Use of the conjugate represented by formula I or the pharmaceutically acceptable salt thereof according to any one of claims 1-5, the RNAi agent according to claim 10, or the pharmaceutical composition according to claim 11 in the manufacture of a medicament for preventing and/or treating a disease, wherein the disease is a neurological disease or disorder, a metabolic disease or disorder, an inflammatory disease or disorder, or cancer.

13. A preparation method for the conjugate represented by formula I or the pharmaceutically acceptable salt thereof according to any one of claims 1-5, being a method I, a method II, or a method III, wherein:
the method I comprises: taking the compound represented by formula II-1A or II-2A according to claim 8 or the compound according to claim 9 as a start and linking nucleoside monomers one by one in the 3'-5' direction in an order in which nucleotides are arranged to give the conjugate;
the method II comprises: taking a universal solid-phase support as a start, linking nucleoside monomers one by one in the 3'-5' direction in an order in which nucleotides are arranged, and then linking the compound represented by formula II-3A or II-4A according to claim 8 to give the conjugate;
the method III comprises: taking the compound represented by formula II-1A or II-2A according to claim 8 as a start, linking nucleoside monomers one by one in the 3'-5' direction in an order in which nucleotides are arranged, and then linking the compound represented by formula II-3A or II-4A according to claim 8 to give the conjugate.
